(19) Europäisches Patentamt
European Patent Office
Office européen des brevets

(11) **EP 4 285 943 B1**

(12) # EUROPEAN PATENT SPECIFICATION

(45) Date of publication and mention of the grant of the patent:
**18.03.2026 Bulletin 2026/12**

(21) Application number: **22746303.1**

(22) Date of filing: **28.01.2022**

(51) International Patent Classification (IPC):
***A61L 15/22*** *(2006.01)* ***A61L 15/18*** *(2006.01)*
***A61L 27/60*** *(2006.01)* ***A61L 27/44*** *(2006.01)*
***A61F 13/02*** *(2024.01)*

(52) Cooperative Patent Classification (CPC):
(C-Sets available)
**A61F 13/0223; A61F 13/00063; A61F 13/0243; A61L 15/18; A61L 15/20; A61L 15/26; A61L 27/047; A61L 27/18; A61L 27/446; A61L 27/60** (Cont.)

(86) International application number:
**PCT/KR2022/001646**

(87) International publication number:
**WO 2022/164277 (04.08.2022 Gazette 2022/31)**

(54) **SILICONE PATCH COMPRISING METAL-ORGANIC FRAMEWORK AND SILICONE COMPOSITION**

SILIKONPFLASTER MIT METALLORGANISCHEM GERÜST UND SILIKONZUSAMMENSETZUNG

PATCH DE SILICONE COMPRENANT UNE STRUCTURE MÉTALLO-ORGANIQUE ET UNE COMPOSITION DE SILICONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2021 KR 20210012474**

(43) Date of publication of application:
**06.12.2023 Bulletin 2023/49**

(73) Proprietors:
- **Seoul National University Hospital**
  **Seoul 03080 (KR)**
- **Sookmyung Women's University Industry-Academic Cooperation Foundation**
  **Seoul 04310 (KR)**

(72) Inventors:
- **HEO, Chan Yeong**
  **Seongnam-si, Gyeonggi-do 13605 (KR)**
- **NAM, Sun Young**
  **Seongnam-si, Gyeonggi-do 13605 (KR)**
- **CHOI, Kyungmin**
  **Seoul 08097 (KR)**
- **SHIN, Dong-Sik**
  **Seoul 04180 (KR)**
- **RYU, Un Jin**
  **Seoul 04310 (KR)**
- **KIM, Seongsoo**
  **Wonju-si, Gangwon-do 26406 (KR)**

(74) Representative: **V.O.**
**P.O. Box 87930**
**2508 DH Den Haag (NL)**

(56) References cited:
**WO-A1-2019/235742**
**WO-A2-2021/118292**
**US-A1- 2009 104 252**
**US-A1- 2011 282 436**
**US-A1- 2017 274 097**
**US-A1- 2018 236 122**
**US-A1- 2019 358 618**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **NAJMIDDINOV BAKHTIYOR: "The effect of silicone patch containing metal-organic framework on the suppression of Hypertrophic scar", MASTER'S THESIS OF GRADUATE SCHOOL OF SEOUL NATIONAL UNIVERSITY, 1 February 2021 (2021-02-01), XP055954210, Retrieved from the Internet <URL:https://s-space.snu.ac.kr/handle/10371/176120> [retrieved on 20220823]**


(52) Cooperative Patent Classification (CPC): (Cont.)

C-Sets
**A61L 27/18, C08L 83/04;**
**A61L 27/446, C08L 83/04**

## Description

### Technical Field

Disclosed herein is a patch comprising a metal-organic framework, specifically a silicone patch comprising a metal-organic framework and a silicone composition.

[National research and development project supporting the present invention]
[Task serial number] 1711112838
[Task number] 2019R1A2C4069764
[Government department] Ministry of Science and ICT
[Task management (specialized) institute] National Research Foundation of Korea
[Research project] Personal basic research (Ministry of Science and ICT) (R&D)
[Research task] Development of high-efficiency and high-stability optical, organic and electrocatalysts through synthesis of metal-organic-unit particles having molecular catalysts
[Contribution ratio] 1/1
[Task performing institute] Sookmyung Women's University
[Research period] March 01, 2020 - February 28, 2021

### Background Art

The skin is composed of three major layers: epidermis, dermis, and subcutaneous fat, and the skin tissue of each layer is composed of various cells that are in charge of various functions of the skin and substances surrounding them. When the skin is damaged, the damaged site is invaded by bacteria and external toxins, and inflammation may be induced by the skin's defense function against this.
Wound healing is a tissue repair process as a tissue response to damaged skin, and is a complex biological process including chemotaxis, cell differentiation and replication, lipoprotein synthesis, angiogenesis, and the like, and various factors are involved.
In the tissue repair process for damaged skin, cell migration process to repair damaged skin site works in combination with cell proliferation. In other words, in order to restore damaged skin tissue, skin cell generation is activated and newly generated cells migrate to the damaged site to form new skin tissue. Therefore, for wound healing, it is essential to use a drug that promotes the regeneration and proliferation of skin cells and regulates the migration of generated skin cells to damaged skin part.
Meanwhile, wound treatment using an appropriate patch is essential in order to rapidly treat wounds and minimize various secondary side effects. As conventional treatment for skin wounds such as burns, trauma, cuts, and decubitus ulcers, a skin wound is treated by applying disinfectant to the wound and covering the wound site with an absorbent material such as gauze or cotton to absorb exudate from the cut and dry the wound site. However, as a result of recent studies, it has been demonstrated that maintaining the wound site in an appropriately moist environment is rather effective for cut treatment, and wound patches of various shapes and materials have been developed and marketed.
For example, WO2019/235742 A1 describes an antibacterial silicone patch containing MOF. The concept of a silicone patch showing excellent wound healing effect has not been disclosed herein.
Under this background, the present inventors have conducted studies on a material that can be produced into a patch, particularly a silicone patch while having an excellent wound healing effect, and completed the present invention.

[Related Art]

[Patent Literature]

1. CN 109111575 A
2. CN 1083555166 A
3. CN 107823220 A

### Disclosure

## Technical Problem

In one aspect, an object of the present invention is to provide a patch, in particular a silicone patch comprising a metal-organic framework for skin cell regeneration.
In another aspect, an object of the present invention is to provide a patch, in particular a silicone patch comprising a metal-organic framework for wound healing.
In still another aspect, an object of the present invention is to provide artificial skin comprising a metal-organic framework.

## Solution to Problem

In one aspect, the present invention provides a patch for skin cell regeneration or wound healing comprising a metal-organic framework (MOF) according to claims 2 and 3.
The present invention provides a silicone patch comprising a metal-organic framework (MOF) and a silicone composition.
In another aspect, the present invention provides artificial skin comprising a metal-organic framework (MOF).
In the patch, silicone patch and/or artificial skin for skin cell regeneration or wound healing described above, the metal-organic framework contains one or more metal ions selected from the group consisting of Al and Zr and one or more organic ligands selected from the group consisting of 4,4'-biphenyldicarboxilic acid, benzene-1,4-dicarboxylic acid, 9,10-anthracenedicarboxylic acid, biphenyl-3,3,5,5'-tetracarboxylic acid, biphenyl-3,4',5-tricarboxylic acid, 5-bromoisophthalic acid, 5-cyano-1,3-benzenedicarboxylic acid, 2,2-diamino-4,4'-stilbenedicarboxylic acid, 2,5-diaminoterephthalic acid, 1,1,2,2-tetra(4-carboxylphenyl)ethylene, 2,5-dihydroxyterephthalic acid, 2,2-dinitro-4,4-stilbenedicarboxylic acid, 5-ethynyl-1,3-benzenedicarboxylic acid, 2-hydroxyterephthalic acid, 2,6-naphthalenedicarboxylic acid, 1,2,4,5-tetrakis(4-carboxyphenyl)benzene, 4,4,4"-s-triazine-2,4,6-triyl-tribenzoic acid, 1,3,5-tricarboxybenzene, 1,4,7,10-tetraazacyclododecane-N,N',N",N"'-tetraacetic acid, 1,3,5-tris(4-carboxy[1,1'-biphenyl]-4-yl)benzene, and 1,3,5-triscarboxyphenylethynylbenzene.
In the patch, silicone patch and/or artificial skin for skin cell regeneration or wound healing described above, the metal-organic framework may be MOF-808 (Zr6O4(OH)4(BTC)2(HCOO)6).
In the silicone patch described above, the metal-organic framework may be contained at 0.01% to 10% by weight based on the total weight of the metal-organic framework and the silicone composition.
In the silicone patch described above, the metal-organic framework may be dispersed in the silicone composition.
In the silicone patch described above, the silicone patch may be for skin cell regeneration and/or wound healing.
In the use for wound healing described above, the wound may be any one or more selected from the group consisting of cuts, non-healing traumatic wounds, tissue destruction by irradiation, burns, abrasions, lacerations, avulsion wounds, penetrating wounds, gunshot wounds, incisions, burns, frostbite, skin ulcers, dry skin, keratosis, cracking, tearing, dermatitis, surgical or vascular disease wounds, bruises, corneal wounds, decubitus ulcers, bedsore, chronic ulcers, postoperative suture sites, spinal injury wounds, gynecological wounds, chemical wounds, and acne.
In the use for wound healing described above, the wound may be a hypertrophic scar.
The patch, silicone patch and/or artificial skin for skin cell regeneration or wound healing described above may satisfy the following characteristics:

(i) a decrease in hypertrophic scar (HS) at a wound site by 25% to 75% after attachment of the patch, silicone patch or artificial skin compared to HS before attachment;
(ii) a decrease in scar elevation index (SEI) at a wound site by 30% to 40% after attachment of the patch, silicone patch or artificial skin compared to SEI before attachment;
(iii) a decrease in epidermal thickness at a wound site by 25% to 75% after attachment of the patch, silicone patch or artificial skin compared to the epidermal thickness before attachment;
(iv) a decrease in density of collagen fibers at a wound site by 10% to 30% after attachment of the patch, silicone patch or artificial skin compared to the density of collagen fibers before attachment;
(v) a decrease in expression level of $\alpha$-smooth muscle action ($\alpha$-SMA) at a wound site by 30% to 50% after attachment of the patch, silicone patch or artificial skin compared to $\alpha$-SMA before attachment;
(vi) a decrease in expression level of collagen I (Col-I) at a wound site by 20% to 40% after attachment of the patch, silicone patch or artificial skin compared to the expression level of Col-I before attachment; and
(vii) a decrease in expression level of transforming growth factor-$\beta$ (TGF-$\beta$) at a wound site by 10% to 30% after attachment of the patch, silicone patch or artificial skin compared to the expression level of TGF-$\beta$ before attachment.

In the artificial skin described above, the artificial skin may further comprise a silicone composition.

## Advantageous Effects of the Invention

The patch, silicone patch or artificial skin according to the present invention has wound healing properties such that the scar area is reduced, the epidermal thickness and collagen density decrease, and the expression levels of α-SMA, collagen I, and TGF-β, which are biomarkers related to wound healing, decrease when attached to scars on the skin. In particular, the patch, silicone patch or artificial skin has an excellent wound healing effect to reduce the scar area by about 50% compared to a control group not containing a metal-organic framework, and can be used as a patch, in particular a silicone patch for cell regeneration or skin wound healing, and furthermore, can be used as artificial skin.

## Brief Description of Drawings

FIGS. 1A to 1C are results of scanning a metal-organic framework according to Example of the present invention, FIG. 1A is a photograph of the metal-organic framework powder taken using a digital camera, FIG. 1B is a photograph of the metal-organic framework taken using a scanning electron microscope, and FIG. 1C is a graph comparing X-ray diffraction patterns of a metal-organic framework (black) prepared in Example of the present invention and a simulated metal-organic framework (grey);

FIGS. 1D to 1F are results of photographing a silicone patch containing a metal-organic framework according to Example of the present invention, FIG. 1D is a photograph of the silicone patch taken using a digital camera, and FIGS. 1E and 1F are photographs of the silicone patch taken from the top (FIG. 1E) and the side (FIG. 1F) using a scanning electron microscope;

FIGS. 2A and 2B are diagrams related to an animal experiment to attach a silicone patch containing a metal-organic framework according to Example of the present invention; FIG. 2A is a photograph taken of anesthetizing a rabbit for the experiment, and FIG. 2B is a photograph of three wounds made in the rabbit's left ear;

FIG. 3A is photographs of the scar sites in a negative control (Control (Group 1)), a positive control (Silicone patch treated (Group 2)) to which a silicone patch not containing a metal-organic framework is attached, and a test group (Silicone patch+0.2% MOF treated (Group 3)) to which a silicone patch containing a metal-organic framework according to Example of the present invention is attached, and FIG. 3B is a graph illustrating the average hypertrophic scar area in each of the negative control (Control), positive control (MOF 0%), and test group (MOF 0.2%);

FIGS. 4A and 4B are diagrams illustrating the scar elevation index (SEI) measured according to Example of the present invention, where "a" is the height from the peak of hypertrophic scar to cartilage and "b" is the height from normal skin surface to cartilage;

FIG. 5 is a graph comparing the scar elevation index (SEI) measured in each of a negative control (control), a positive control (MOF 0%) to which a silicone patch not containing a metal-organic framework is attached, and a test group (MOF 0.2%) to which a silicone patch containing a metal-organic framework according to Example of the present invention is attached according to Example of the present invention, where the statistical difference is set at $p < 0.05$, the displayed value is the mean ± SD (n = 3 repetitions), and a, b, and c are statistically different;

FIG. 6A is photographs of epidermis stained with H&E and taken according to Example of the present invention in each of a negative control (Control (Group 1)), a positive control (Positive (Group 2)) to which a silicone patch not containing a metal-organic framework is attached, and a test group (MOF treated (Group 3)) to which a silicone patch containing a metal-organic framework according to Example of the present invention is attached, and FIG. 6B is a graph comparing the epidermal thickness measured in each of the negative control (Control), positive control (MOF 0%), and test group (MOF 0.2%), where the displayed value is the mean ± SD (n = 5 repetitions) and a, b, and c are statistically different $p < 0.05$;

FIG. 6C is photographs of epidermis undergone Masson's Trichrome staining and taken according to Example of the present invention in a negative control (Control (Group 1)), a positive control (Positive (Group 2)) to which a silicone patch not containing a metal-organic framework is attached, and a test group (MOF treated (Group 3)) to which a silicone patch containing a metal-organic framework according to Example of the present invention is attached, and FIG. 6D is a graph comparing the collagen density measured in each of the negative control (Control), positive control (MOF 0%), and test group (MOF 0.2%), where the displayed value is the mean ± SD (n = 5 repetitions) and a, b, and c are statistically different $p < 0.05$; and

FIGS. 7A and 7B are graphs comparing western blot results and expression levels of α-SMA, collagen I, and TGF-β measured according to Example of the present invention in a negative control (Control), a positive control (Positive in FIG. 7A, MOF 0% in FIG. 7B) to which a silicone patch not containing a metal-organic framework is attached, and a test group (MOF in FIG. 7A, MOF 0.2% in FIG. 7B) to which a silicone patch containing a metal-organic framework according to Example of the present invention is attached, where the displayed value is the mean ± SD (n = 3 repetitions), significance is set at $p < 0.05$, and a, b, and c indicate significant differences.

## Mode for Invention

Hereinafter, the present invention will be described in detail.

In the present specification, the term "metal-organic framework (MOF)" refers to a porous material in which a metal cluster and an organic linker (or organic bridging ligand) are connected by a coordinate bond to form a three-dimensional structure, and various MOFs can be formed depending on the selection of metal ions and organic ligands. The MOF is characterized by being porous with empty spaces in the structure, and the pore size, porosity, 3D structure, surface area, and the like can be variously designed depending on the kind and bonding of metal ions and organic ligands constituting the MOF. The MOF has advantages of not only having a greatly large surface area due to this porosity but also having an open pore structure so that a larger amount of molecules or solvents can migrate therethrough compared to other previously known porous materials, and having a large number of active sites to maximize the efficiency when used as a catalyst or gas reservoir. The MOF is not easily deformed at high temperatures and has a hard skeleton so that the chemical and thermal stability thereof is excellent.

In the present specification, the "skin" refers to an organ covering the outside of an organism, is composed of epidermis, dermis, and subcutaneous fat layer, and is the broadest concept that includes the scalp and hair as well as tissues covering the outside of the face or the entire body.

In one aspect, the present invention provides a patch for skin cell regeneration or wound healing comprising a metal-organic framework (MOF) according to claim 2 or 3.

The present invention provides a silicone patch comprising a metal-organic framework (MOF) and a silicone composition.

In another aspect, the present invention provides artificial skin comprising a metal-organic framework (MOF).

The metal-organic framework contains one or more metal ions selected from the group consisting of Al and Zr and one or more organic ligands selected from the group consisting of 4,4'-biphenyldicarboxilic acid, benzene-1,4-dicarboxylic acid, 9,10-anthracenedicarboxylic acid, biphenyl-3,3,5,5'-tetracarboxylic acid, biphenyl-3,4',5-tricarboxylic acid, 5-bromoisophthalic acid, 5-cyano-1,3-benzenedicarboxylic acid, 2,2-diamino-4,4'-stilbenedicarboxylic acid, 2,5-diaminoterephthalic acid, 1,1,2,2-tetra(4-carboxylphenyl)ethylene, 2,5-dihydroxyterephthalic acid, 2,2-dinitro-4,4-stilbenedicarboxylic acid, 5-ethynyl-1,3-benzenedicarboxylic acid, 2-hydroxyterephthalic acid, 2,6-naphthalenedicarboxylic acid, 1,2,4,5-tetrakis(4-carboxyphenyl)benzene, 4,4,4"-s-triazine-2,4,6-triyl-tribenzoic acid, 1,3,5-tricarboxybenzene, 1,4,7,10-tetraazacyclododecane-N,N',N",N"'-tetraacetic acid, 1,3,5-tris(4-carboxy[1,1'-biphenyl]-4-yl)benzene, and 1,3,5-triscarboxyphenylethynylbenzene. The metal-organic framework may be specifically MOF-808 (Zr6O4(OH)4(BTC)2(HCOO)6), MOF-801 (Zr6O4(OH)4(fumarate)6), Zr6O6(BDC)6, Zr6O6(NH-2-BDC)6, Zr6O6(OH)4(BPDC)6, Al3O(OH)(H2O)2(BDC-NH2)3 and Al(OH)(BDC), more specifically MOF-808 (Zr6O4(OH)4(BTC)2(HCOO)6), but the kind thereof is not limited as long as it is a metal-organic framework that ensures safety when applied to a wound site on the skin, can be used for wound healing, and further has safety, stability and wound healing properties to be contained as an active ingredient of a silicone patch.

In exemplary embodiments, the metal-organic framework may be contained at 0.01% to 10% by weight based on the total weight of the metal-organic framework and the silicone composition. Specifically, the metal-organic framework may be contained at 0.01% by weight or more, 0.05% by weight or more, 0.1% by weight or more, 0.11% by weight or more, 0.12% by weight or more, 0.13% by weight or more, 0.14% by weight or more, 0.15% by weight or more, 0.16% by weight or more, 0.17% by weight or more, 0.18% by weight or more, 0.19% by weight or more, 0.2% by weight or more, 0.25% by weight or more, 0.3% by weight or more, 0.35% by weight or more, 0.4% by weight or more, 0.45% by weight or more, 0.5% by weight or more, 0.55% by weight or more, 0.6% by weight or more, 0.65% by weight or more, 0.7% by weight or more, 0.71% by weight or more, 0.72% by weight or more, 0.73% by weight or more, 0.74% by weight or more, 0.75% by weight or more, 0.76% by weight or more, 0.77% by weight or more, 0.78% by weight or more, 0.79% by weight or more, 0.8% by weight or more, 0.85% by weight or more, 0.9% by weight or more, 0.95% by weight or more, 1% by weight or more, 5% by weight or more or 10% by weight or more based on the total weight of the metal-organic framework and the silicone composition, and the metal-organic framework may be contained at 10% by weight or less, 5% by weight or less, 1% by weight or less, 0.95% by weight or less, 0.9% by weight or less, 0.89% by weight or less, 0.88% by weight or less, 0.87% by weight or less, 0.86% by weight or less, 0.85% by weight or less, 0.84% by weight or less, 0.83% by weight or less, 0.82% by weight or less, 0.81% by weight or less, 0.8% by weight or less, 0.75% by weight or less, 0.7% by weight or less, 0.65% by weight or less, 0.6% by weight or less, 0.55% by weight or less, 0.5% by weight or less, 0.45% by weight or less, 0.4% by weight or less, 0.35% by weight or less, 0.3% by weight or less, 0.29% by weight or less, 0.28% by weight or less, 0.27% by weight or less, 0.26% by weight or less, 0.25% by weight or less, 0.24% by weight or less, 0.23% by weight or less, 0.22% by weight or less, 0.21% by weight or less, 0.2% by weight or less, 0.15% by weight or less, 0.1% by weight or less, 0.05% by weight or less or 0.01% by weight based on the total weight of the metal-organic framework and the silicone composition, but the content thereof is not limited as long as it is a content so that the metal-organic framework is cytotoxic enough to ensure safety when applied to a wound site on the skin, can be used for wound healing, and further has safety, stability and wound healing properties to be contained as an active ingredient in a silicone patch.

In exemplary embodiments, the silicone composition may contain 10 to 100 parts by weight of a polysiloxane having a vinyl group; 5 to 40 parts by weight of dimethyl silicone oil; 1 to 50 parts by weight of a polysiloxane having a Si-H bond; 0.5 to 20 parts by weight of a silicone resin; and 0.1 to 5 parts by weight of a platinum catalyst. Specifically, the silicone composition may contain 70 to 90 parts by weight of a polysiloxane having a vinyl group; 10 to 20 parts by weight of dimethyl silicone oil; 5 to 15 parts by weight of a polysiloxane having a Si-H bond; 0.5 to 20 parts by weight of a silicone resin; and 0.3 to 0.6 parts by weight of a platinum catalyst. More specifically, the silicone composition may further contain 1 to 100 parts by weight of an absorbent material, still more specifically 2 to 60 parts by weight of an absorbent material. A composition containing an absorbent material as described above is cured to form a hydrocolloid dressing material. The silicone composition may further contain 0.01 to 20 parts by weight of a drug, if necessary.

In exemplary embodiments, the polysiloxane having a vinyl group is a gel-like silicone, and may be prepared by crosslinking siloxane chains by an addition reaction with a polysiloxane having a Si-H bond in the presence of a platinum catalyst. The specific reaction scheme is as Reaction Scheme 1 below.

[Reaction Scheme 1]

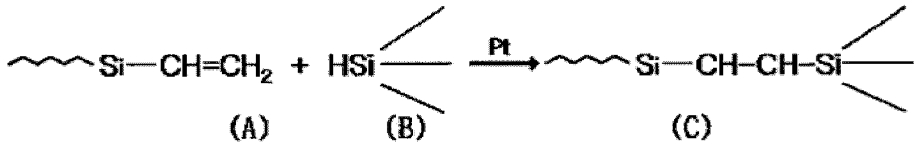


(A) (B) (C)

In exemplary embodiments, the dimethyl silicone oil may be represented by the following Chemical Formula 1, and has various viscosities from low viscosity to high viscosity so that dimethyl silicone oil having a proper viscosity may be selected and used depending on the purpose of use. In one aspect of the present invention, the dimethyl silicone oil is added for the purpose of viscosity control, and may be selected from those having a viscosity specifically in a range of 0.65 to 100,000 cps/25°C, more specifically in a range of 0.65 to 20 cps/25°C and used.

[Chemical Formula 1]

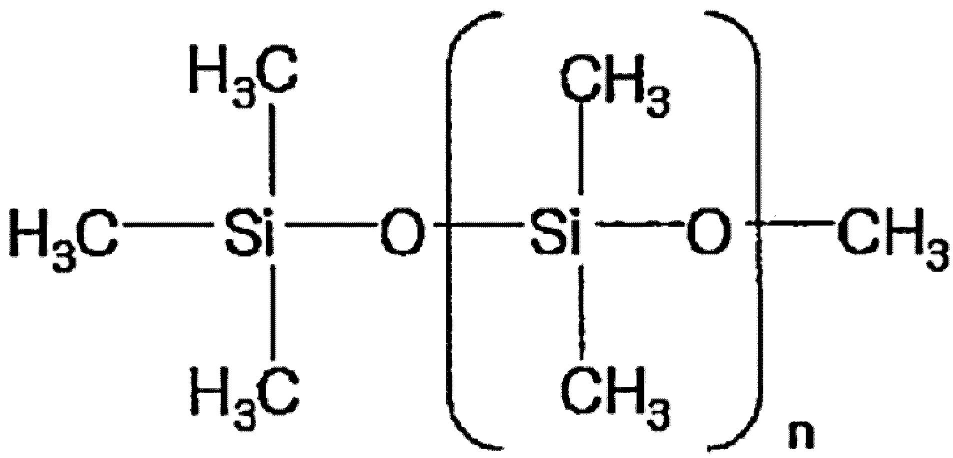

In exemplary embodiments, the polysiloxane having a Si-H bond is a compound represented by (B) in Reaction Scheme 1, and undergoes a crosslinking reaction with the polysiloxane having a vinyl group. In one aspect of the present invention, the polysiloxane having a Si-H bond may be specifically TSF484TM from GE Silicone, SP6000PTM from KCC, KF-99TM from Shin-Etsu, and the like, but is not limited thereto.

In exemplary embodiments, the silicone resin (silicone resin) has a change in adhesive strength depending on the amount added and the content of OH, and can be used by appropriately adjusting or selecting the amount to be added by those skilled in the related art. Specifically, the silicone resin of one aspect of the present invention may have a number average molecular weight (Mn) in a range of 1,000 to 10,000, and the molar ratio of "M" unit to "Q" unit in the general formula represented by the following Chemical Formula 2 may be 0.5:1 to 1.3:1. More specifically, the silicone resin of one aspect of the present invention may have a number average molecular weight (Mn) in a range of 3,000 to 7,000, and the molar ratio of "M" unit to "Q" unit may be 0.5:1 to 1.0:1. The following Chemical Formula 2 is a general formula of silicone resin, where R is a functional or non-functional group.

[Chemical Formula 2]

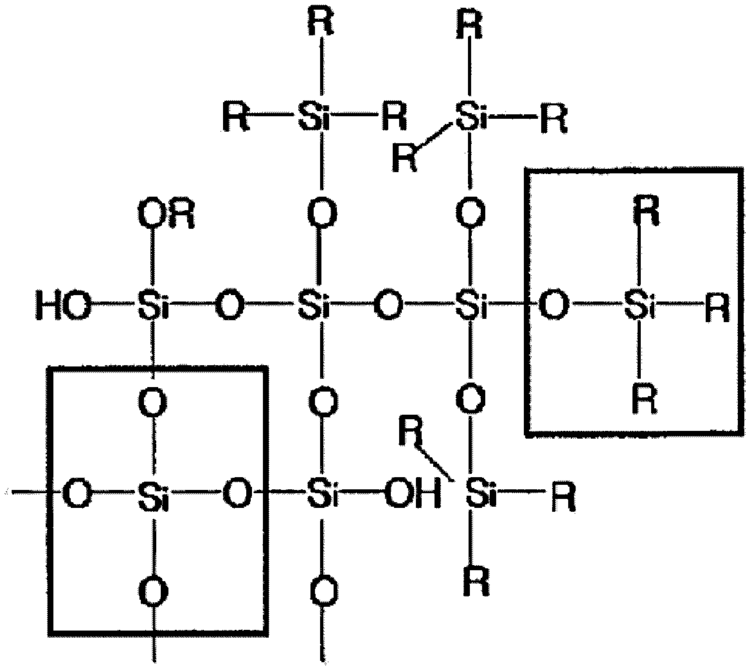


In exemplary embodiments, the platinum catalyst is used as a curing accelerator. Specifically, (ethylene) bis(triphenylphosphine)platinum (Pt{P(C6H5)3}2(C2H4)) may be used alone, or one or more of phosphate-based platinum compounds may be used in mixture. Specific examples of the phosphate-based platinum compounds include Pt{P(OC6H5)3}4, Pt{P(C6H5)3}4, PT{P(C4H9)3}4, and Pt{P(OCH3)3}4. The platinum catalyst may be used after being dispersed in isopropyl alcohol or the like.

In exemplary embodiments, the silicone composition may have a viscosity in a range of 5,000 to 40,000 cps/25°C. In a case where the viscosity is less than 5,000 cps/25°C, it is difficult to keep the thickness constant until the silicone composition is cured with heat after being applied in a certain thickness. In a case where the viscosity is 40,000 cps/25°C or more, it is difficult to coat a breathable waterproof polyurethane film layer with the silicone composition. More specifically, the silicone composition may have a viscosity of 20,000 to 30,000 cps/25°C.

It is noted that 1 cps converts to 1 mPa.s.

In exemplary embodiments, the metal-organic framework may be dispersed in the silicone composition.

In exemplary embodiments, the silicone patch may be for skin cell regeneration. The skin cell regeneration may be to promote skin cell proliferation or promote skin cell migration.

In exemplary embodiments, the silicone patch may be for wound healing. In the present specification, the wound means to encompass all that a living body is damaged, may refer to a state in which a living body is damaged, and is also referred to as a scar or a cut.

The wound may be any one or more selected from the group consisting of cuts, non-healing traumatic wounds, tissue destruction by irradiation, burns, abrasions, lacerations, avulsion wounds, penetrating wounds, gunshot wounds, incisions, burns, frostbite, skin ulcers, dry skin, keratosis, cracking, tearing, dermatitis, surgical or vascular disease wounds, bruises, corneal wounds, decubitus ulcers, bedsore, chronic ulcers, postoperative suture sites, spinal injury wounds, gynecological wounds, chemical wounds, and acne, but is not limited thereto. Alternatively, the wound may be a hypertrophic scar. The hypertrophic scar refers to an irregular wound healing process clinically defined by excessive generation of collagen and excessive deposition of extracellular matrix (ECM) due to deep skin wounds [G. Gauglitz, Management of keloids and hypertrophic scars: current and emerging options, Clin. Cosmet. Investig. Dermatol. (2013) 103. https://doi.org/10.2147/ccid.s35252]. When the skin is damaged, a scar is formed to heal the defect, and linear collagen is deposited so that the skin is not damaged. The increase in hypertrophic scars is related to scar growth, increases in epidermal thickness, increases in collagen density, and expression of specific biomarkers. In exemplary embodiments, wounds, cuts and scars may be used interchangeably. According to Example of the present invention, in a case where a silicone patch containing a metal-organic framework and a silicone composition according to one aspect of the present invention is attached to the ear of a rabbit whose skin has been cut, the wound, specifically hypertrophic scar is healed such that the scar area is reduced, the scar elevation index (SEI) and the epidermal thickness decrease, and the

collagen fibers are smoothly formed as time elapses. Hence, it has been confirmed that the silicone patch containing a metal-organic framework and a silicone composition according to Example of the present invention has wound healing properties and can be used as a silicone patch or artificial skin for wound healing (Experimental Examples 2 and 3).

In exemplary embodiments, the patch, silicone patch or artificial skin may have one or more characteristics selected from the group consisting of (i) a decrease in hypertrophic scar (HS) at a wound site by 25% to 75% after attachment of the patch, silicone patch or artificial skin compared to HS before attachment; (ii) a decrease in scar elevation index (SEI) at a wound site by 30% to 40% after attachment of the patch, silicone patch or artificial skin compared to SEI before attachment; (iii) a decrease in epidermal thickness at a wound site by 25% to 75% after attachment of the patch compared to the epidermal thickness before attachment; (iv) a decrease in density of collagen fibers at a wound site by 10% to 30% after attachment of the patch, silicone patch or artificial skin compared to the density of collagen fibers before attachment; (v) a decrease in expression level of α-smooth muscle action (α-SMA) at a wound site by 30% to 50% after attachment of the patch, silicone patch or artificial skin compared to α-SMA before attachment; (vi) a decrease in expression level of collagen I (Col-I) at a wound site by 20% to 40% after attachment of the patch, silicone patch or artificial skin compared to the expression level of Col-I before attachment; and (vii) a decrease in expression level of transforming growth factor-β (TGF-β) at a wound site by 10% to 30% after attachment of the patch compared to the expression level of TGF-β before attachment.

Specifically, in an embodiment, the hypertrophic scar (HS) at the wound site may be reduced by 25% to 75% after attachment of the patch, silicone patch, or artificial skin compared to that before attachment of the patch, silicone patch, or artificial skin, more specifically, the hypertrophic scar may be reduced by 25% or more, 30% or more, 35% or more, 40% or more, 45% or more, 50% or more, 55% or more, 60% or more, 65% or more, or 70% or more and by 75% or less, 70% or less, 65% or less, 60% or less, 55% or less, 50% or less, 45% or less, 40% or less, 35% or less, or 30% or less after attachment of the patch, silicone patch, or artificial skin compared to that before attachment.

Specifically, in an embodiment, the scar elevation index (SEI) at the wound site may be reduced by 30% to 40% after attachment of the patch, silicone patch, or artificial skin compared to that before attachment of the patch, silicone patch, or artificial skin, more specifically, the scar elevation index (SEI) at the wound site may be reduced by 30% or more, 31% or more, 32% or more, 33% or more, 34% or more, 35% or more, 36% or more, 37% or more, 38% or more, or 39% or more and by 40% or less, 39% or less, 38% or less, 37% or less, 36% or less, 35% or less, 34% or less, 33% or less, 32% or less, or 31% or less after attachment of the patch, silicone patch, or artificial skin compared to that before attachment.

In a non-limiting example, according to Example of the present invention, in a case where a silicone patch containing a metal-organic framework is attached to a wound site, the scar elevation index is further reduced by 34.6% and 25.6%, respectively, compared to that in a negative control to which a silicone patch is not attached and that in a positive control to which a silicone patch not containing a metal-organic framework is attached, and it has been thus confirmed that the silicone patch according to one aspect of the present invention has an excellent wound healing properties (Experimental Example 2).

Specifically, in an exemplary embodiment, the epidermal thickness at the wound site may be reduced by 25% to 75% after attachment of the patch, silicone patch, or artificial skin compared to that before attachment of the patch, silicone patch, or artificial skin, more specifically, the epidermal thickness may be reduced by 25% or more, 30% or more, 35% or more, 40% or more, 45% or more, 50% or more, 55% or more, 60% or more, 65% or more, or 70% or more and by 75% or less, 70% or less, 65% or less, 60% or less, 55% or less, 50% or less, 45% or less, 40% or less, 35% or less, or 30% or less after attachment of the patch, silicone patch, or artificial skin compared to that before attachment.

Specifically, in an exemplary embodiment, the density of collagen fibers at the wound site may be reduced by 10% to 30% after attachment of the patch, silicone patch, or artificial skin compared to that before attachment of the patch, silicone patch, or artificial skin, more specifically, the density of collagen fibers may be reduced by 10% or more, 12% or more, 14% or more, 16% or more, 18% or more, 20% or more, 22% or more, 24% or more, 26% or more, 28% or more, or 29% or more and by 30% or less, 28% or less, 26% or less, 24% or less, 22% or less, 20% or less, 18% or less, 16% or less, 14% or less, or 12% or less after attachment of the patch, silicone patch, or artificial skin compared to that before attachment.

In a non-limiting example, according to Example of the present invention, in a case where a silicone patch containing a metal-organic framework is attached to a wound site, the epidermal thickness and the collagen density more greatly decrease compared to those in a negative control to which a silicone patch is not attached and those in a positive control to which a silicone patch not containing a metal-organic framework is attached, and it has been thus confirmed that the silicone patch according to one aspect of the present invention has an excellent effect of healing wounds, specifically hypertrophic scars, such as smooth formation of collagen fibers and decreases in epidermal thickness (Experimental Example 3).

Specifically, in an exemplary embodiment, the expression level of α-SMA at the wound site may be reduced by 30% to 50% after attachment of the patch, silicone patch, or artificial skin compared to that before attachment of the patch, silicone patch, or artificial skin, more specifically, the expression level of α-SMA may be reduced by 30% or more, 32% or more, 34% or more, 36% or more, 38% or more, 40% or more, 42% or more, 44% or more, 46% or more, or 48% or more

and by 50% or less, 48% or less, 46% or less, 44% or less, 42% or less, 40% or less, 38% or less, 36% or less, 34% or less, or 32% or less after attachment of the patch, silicone patch, or artificial skin compared to that before attachment. Fibroblasts in the skin at a hypertrophic scar site express α-SMA, and an effect of healing wounds is acquired by regulating the activity of fibroblasts through decreases in the expression level of α-SMA.

Specifically, in an exemplary embodiment, the expression level of collagen I at the wound site may be reduced by 20% to 40% after attachment of the patch, silicone patch, or artificial skin compared to that before attachment of the patch, silicone patch, or artificial skin, more specifically, the expression level of collagen I may be reduced by 20% or more, 22% or more, 24% or more, 26% or more, 28% or more, 30% or more, 32% or more, 34% or more, 36% or more, or 38% or more and by 40% or less, 38% or less, 36% or less, 34% or less, 32% or less, 30% or less, 28% or less, 26% or less, 24% or less, or 22% or less after attachment of the patch, silicone patch, or artificial skin compared to that before attachment. Myofibroblasts stimulated in wounds, specifically hypertrophic scarred skin, are involved in repair by collecting a large amount of extracellular matrix proteins, and this may play a role in healing wound contraction [A. V Shinde, C. Humeres, N.G. Frangogiannis, The role of α-smooth muscle actin in fibroblast-mediated matrix contraction and remodeling, Biochim. Biophys. Acta - Mol. Basis Dis. 1863 (2017) 298-309. https://doi.org/10.1016/j.bbadis.2016.11.006]. Myofibroblasts regulate the formation of collagen fibers in the remodeling stage and promote the degradation of collagen to reduce the adhesion of myofibroblasts by a cell death mechanism [M, Xue, C.J. Jackson, Extracellular matrix reorganization during wound healing and its impact on abnormal scarring, Adv. Wound Care. 4(3) (2015) 119-136. https://doi.org/10.1089/wound.2013.0485], and fibroblasts in the skin at a hypertrophic scar site increase the level of collagen I synthesis [V. Moulin, S. Larochelle, C. Langlois, I. Thibault, C.A. Lopez-Valle, M. Roy, Normal skin wound and hypertrophic scar myofibroblasts have differential responses to apoptotic inductors. J. Cell Physiol. 198 (2004) 350. https://doi.org/10.1002/jcp.10415].

Specifically, in an exemplary embodiment, the expression level of TGF-β at the wound site may be reduced by 10% to 30% after attachment of the patch, silicone patch, or artificial skin compared to that before attachment of the patch, silicone patch, or artificial skin, more specifically, the expression level of TGF-β may be reduced by 10% or more, 12% or more, 14% or more, 16% or more, 18% or more, 20% or more, 22% or more, 24% or more, 26% or more, or 28% or more and by 30% or less, 28% or less, 26% or less, 24% or less, 22% or less, 20% or less, 18% or less, 16% or less, 14% or less, or 12% or less after attachment of the silicone patch compared to that before attachment. In a case of being wounded, a hypertrophic scar may be formed by excessive collagen generation and deposition by fibroblasts due to increased activity of TGF-β [M. Shah, D.M. Foreman, M.W. Ferguson, Neutralisation of TGF-beta 1 and TGF-beta 2 or exogenous addition of TGF-beta 3 to cutaneous rat wounds reduces scarring, J. Cell Sci. 108(3) (1995) 985-1002. PMID: 7542672]. TGF-β is released in concert with a specialist matrix protein that promotes fibroblasts to become myofibroblasts [A. Leask, Focal Adhesion Kinase: A Key Mediator of Transforming Growth Factor Beta Signaling in Fibroblasts, Adv. Wound Care. 2 (2013) 247-249. https://doi.org/10.1089/wound.2012.0363].

In a non-limiting example, according to Example of the present invention, in a case where a silicone patch containing a metal-organic framework is attached to a wound site, the expression levels of α-SMA, collagen I, and TGF-β decrease compared to those in a negative control to which a silicone patch is not attached and those in a positive control to which a silicone patch not containing a metal-organic framework is attached, and it has been thus found that the silicone patch according to one aspect of the present invention has an excellent wound healing effect to control and inhibit wounds by regulating the expression levels of anti-inflammatory cytokines and biomarkers of myofibroblasts (Experimental Example 4).

In another aspect, the present invention provides artificial skin comprising a metal-organic framework (MOF) and a silicone composition. The metal-organic framework and the silicone composition are as described above.

In one aspect of the present invention, the metal-organic framework has skin cell regeneration ability and can be contained as an active ingredient in artificial skin.

[Examples]

Hereinafter, the configuration and effect of the present invention will be described in more detail with reference to Examples and Experimental Examples. However, the following Examples and Experimental Examples are provided only for illustrative purposes to aid understanding of the present invention, and the gist and scope of the present invention are not limited thereto.

**[Example 1] Preparation of MOF-808 as metal-organic framework**

In order to prepare MOF-808 as a metal-organic framework (MOF) contained as an active ingredient in a cell regeneration composition, the following process was performed.

Specifically, in a 500 mL glass vial, 4.8 g (15 mmol) of $ZrOCl\cdot8H2O$ (99%, Junsei) as a metal moiety was dissolved 112.5 mL of N,N'-dimethylformamide (DMF) (99.8%, Sigma-Aldrich), and then 225 mL of formic acid (96%, Sigma-Aldrich)

was further mixed. With this, 11.25 mL of a DMF solution mixed with 1.05 g (5 mmol) of trimesic acid (95%, Sigma-Aldrich) for preparing a ligand moiety was mixed, and then the reaction was conducted at 130°C for 2 days. Thereafter, this was cooled to room temperature, centrifuged at 6,000 rpm for 10 minutes, and the white solid pellet was then washed with DMF three times. The obtained product was immersed in DMF for 3 days and then sequentially immersed in distilled water and acetone for 3 days. The solvent was replaced with a new solvent every day 3 times a day, and the obtained product was dried and washed to prepare MOF-808, and the prepared MOF-808 was stored in a desiccator.

**[Example 2] Preparation of silicone patch containing MOF-808**

In order to prepare a silicone patch containing MOF-808, a silicone adhesive surface composition and a protective film were prepared through the following processes, respectively, and a silicone patch was prepared using the prepared silicone adhesive surface composition and protective film.

First, 0.2 parts by weight of MOF-808 prepared in Example 1 was added to a silicone composition containing 80 parts by weight of a polysiloxane having a vinyl group, 10 parts by weight of dimethyl silicone oil, 10 parts by weight of a polysiloxane having a Si-H bond, 10 parts by weight of a silicone resin, and 0.5 parts by weight of a platinum catalyst, and mixing was performed at a speed of 1,000 to 3,000 RPM using a mechanical stirrer. At this time, the MOF-808 was uniformly dispersed in the silicone composition for about 1 to 2 hours under reduced pressure (< 0.1 MPa) to prepare a silicone adhesive surface composition.

A polyurethane solution was prepared by mixing a breathable waterproof polyurethane resin and solvents (dimethylformamide, ethyl acetate, methylethylketone), and then sheet molding was performed on the release paper to prepare a breathable waterproof polyurethane film layer.

The silicone adhesive surface composition containing a silicone composition in which MOF-808 was uniformly dispersed was uniformly applied to the prepared breathable waterproof polyurethane film to prepare a silicone patch containing MOF-808. At this time, a silicone patch in which a silicone adhesive surface composition not containing MOF-808 was uniformly applied to the silicone protective film was prepared as a control.

The prepared silicone patch has adhesive strength capable of being attached to a wound so that the time attached to the wound is long and the movement is not hindered after attachment, the silicone patch is formed of a film mixed with MOF-808, and the other half of the adhesive surface is composed of a transparent protective film to eliminate external influences.

**[Experimental Example 1] Observation of characteristics of silicone patch containing MOF-808**

The characteristics of MOF-808 prepared in Example 1 and the silicone patch prepared in Example 2 to contain MOF-808 were observed under a scanning electron microscope (SEM) (JSM-7600F, JEOL). The observation was performed in GB LOW mode at WD of 8.0 mm and scanning was performed at 3 kV to detect the surface morphologies of MOF-808 and the silicone sheet. Cross-sectional SEM images were analyzed using a focused ion beam/scanning electron microscope (FIB/SEM, QuantaTM 3D FEG, FEI), and powder X-ray diffraction patterns (PXRD) were traced using a Bruker advance diffractometer D8 (Cu K$\alpha$ Radiation $\alpha$= 1.54056 Å). The samples were stored in silicone holders and continuously scanned at a scan rate of 5°/min (40 KV, 40 mA).

FIGS. 1A to 1C are the results of scanning of MOF-808 prepared in Example 1, and the MOF-808 has been confirmed to be synthesized by a hydrothermal reaction and have high strength and stability. As illustrated in FIG. 1A, MOF-808 had a white powdery texture to be rapidly dispersed in liquid media. In particular, the finely dissociated MOF powder was dried by a freeze-drying process. As illustrated in FIG. 1B, it has been confirmed that the MOF-808 powder has a uniform size (about 600 nm) and a uniform shape (octahedron). Referring to FIG. 1C illustrating the PXRD image of MOF-808, the MOF-808 has a sharp diffraction peak indicating high crystalline quality, and each diffraction peak is related to the simulated pattern, so the sample structure is similar to the predicted one.

The silicone patch was observed under SEM and FIB/SEM to analyze the existence of MOF-808 on the surface and inside of the silicone patch, and the results are as illustrated in FIGS. 1D to 1F. As illustrated in FIG. 1D, the silicone patch is a silicone patch containing the nano-sized MOF-808 and is in the form of a single-sided adhesive film. FIGS. 1E and 1F are SEM images taken from the top and sides of a silicone patch containing MOF-808 at 0.2%. According to FIG. 1E, 0.2% MOF-808 particles seem to be ununiformly dispersed in the silicone patch. However, according to FIG. 1F, it has been confirmed that the MOF-808 particles are uniformly dispersed inside the silicone layer between the silicone matrices when observed from the side. This allows the MOF-808 particles of Example 1 to spread well in and out of the entire silicone patch of Example 2.

[Experimental Example 2] Examination of scar reducing effect by silicone patch containing MOF-808

[Experimental Example 2-1] Animal experiment to attach silicone patch containing MOF-808

An animal experiment was conducted for an experiment to attach the silicone patch prepared in Example 2 to contain MOF-808.

At this time, five rabbits (New Zealand White (NZW) conventional Rabbit) weighing about 3 to 4 kg were used, and the animal experiment was conducted according to the NIH Animal Care and Use Guide (NIH) with the approval (approval number BA-1911-284-086-01) of the Animal Care and Use Committee of the Seoul National University Bundang Hospital Animal Research Center (Seongnam, Gyeonggi, Korea). The NZW conventional Rabbits were females and purchased from Orient Bio (Seongnam, Gyeonggi, Korea).

For the silicone patch attachment experiment, rabbits were anesthetized by injecting ketamine (60 mg/kg) and xylazine (5 mg/kg) (FIG. 2A). When the anesthesia was completed, the surgical site was sufficiently disinfected using betadine, and the surrounding site was also disinfected to prevent infection. Using a biopsy punch, three circular wounds with a diameter of 12 mm were then made on the bare cartilage of the ventral surface of the rabbit's left ear for each of the following three groups (FIG. 2B). At this time, the epidermis, dermis, and perichondrium were removed from each wound using a dermal punch, a surgical blade, and scissors while observing each wound under a dissecting microscope, but the ear cartilage was not damaged. The group was randomly classified into a negative control, a positive control, and a test group, one silicone patch was attached to each animal for the positive control and test group, and the experiment was conducted on a total of five rabbits in three groups.

- Negative control: Group to which silicone patch is not attached
- Positive control: Group to which silicone patch (0% MOF silicone patch) not containing MOF-808 is attached (Wonbiogene Co. Ltd, Korea)
- Test group: Group to which silicone patch (0.2% MOF silicone patch) of Example 2 prepared to contain MOF-808 of Example 1 at 0.2%

After self-healing for 2 weeks, treatment using the MOF-silicone patch was started, and in the case of positive control and test group, the silicone patches were attached every day for 3 weeks and supervised to be hypertrophic scar models (HS models). Rabbits after surgery were supervised according to standard post-operative animal care protocols. Specifically, according to the standard post-operative animal care protocols, after surgery, the ears were wrapped well with a bandage and a collar was placed so that the rabbit could not scratch the ears, and management supervision was faithfully implemented to protect artificial wounds. In order to protect the surgical site, the animals were closely observed, and ketoprofen was injected into 3SC every day, and the animals were bred one per one cage.

Then, a 0.2% MOF silicone patch to be applied to the test group and a 0% MOF silicone patch to be applied to the positive control were prepared to fit the 12 mm scar, respectively. After scarring, the silicone patch was applied to each of the positive control and test group three times for three weeks. Then, while the silicone patch was replaced, a scar image was taken (FIG. 3A) to measure the wound size. Six weeks after scarring, the animals were euthanized using potassium chloride (KCl), and scar-treated tissue samples were collected 35 days later. Then, the scar hyperplasia rate was measured from the collected tissue sections using the software Imgae J (National Institutes of Health, Bethesda, USA), and the results are as illustrated in FIG. 3B.

As illustrated in FIG. 3A, there is observed a distinct red scar with a hard, thick portion in the center in the untreated negative control. It has been confirmed that the positive control and test group treated with a silicone patch have lighter colors and softer scars and that the test group to which the MOF silicone patch is attached has a more paled scar color and a skin color more similar to normal skin color compared to the positive control.

As illustrated in FIG. 3B, it has been confirmed that the hypertrophic scar (HS) is reduced by 11.1% in the positive control (MOF 0% in FIG. 3B) and by 53.5% in the test group (MOF 0.2% in FIG. 3B) to which the MOF silicone patch is attached (each $p < 0.05$) compared to the negative control (control in FIG. 3B).

Through this, it has been found that the silicone patch containing MOF according to one aspect of the present invention has an excellent wound healing effect to reduce wounds, specifically, hypertrophic scars.

**[Experimental Example 2-2] Measurement of scar elevation index** (SEI)

In Experimental Example 2-1, it has been visually confirmed that the silicone patch containing MOF according to one aspect of the present invention has an excellent wound healing effect, and the following experiment was conducted to confirm the effect more objectively by measuring the scar elevation index (SEI).

The tissue obtained in Experimental Example 2-1 was fixed in 10% neutral buffered formalin (NBF) at 4°C for 24 hours. Next, each paraffin-embedded sample was stained with hematoxylin and eosin (H&E) according to standard protocols, images were taken using an optical microscope (Carl Zeiss, Germany) at 10X magnification, and the scar elevation index (SEI) and epidermal thickness were evaluated. At this time, before the experiment, the hypertrophy degree

of each scar was indicated by SEI and was generally measured at 40X magnification using the tissue sections. SEI was calculated by Equation 1 below, and FIG. 4 is a diagram illustrating SEI. The measurement was performed three times, and the average value was recorded. The SEI was graded on a scale of 1 to 4, and is distorted as the number of collagens increases, the vascularity increases, and the number of inflammatory cells increases.

[Equation 1]

$$SEI = a/b$$

(a: height from peak of hypertrophic scar to cartilage, b: height from normal skin surface to cartilage)

The wound sizes in the negative control, the positive control, and the test group to which a MOF silicone patch is attached are different from one another. As illustrated in FIG. 5, it has been confirmed that the SEI in the test group (MOF 0.2% in FIG. 5) is further reduced by 34.6% compared to that in the negative control (control in FIG. 5), and by 25.6% compared to that in the positive control (MOF 0% in FIG. 5).

Through this, it has been found that the silicone patch containing MOF according to one aspect of the present invention has a superior wound healing effect compared to a silicone patch not containing MOF.

**[Experimental Example 3] Observation of changes in epidermal thickness and collagen density**

The following experiment was conducted to quantitatively measure the changes in epidermal thickness and collagen density depending on the attachment of a silicone patch on scars.

First, the tissues obtained in Experimental Example 2-1 were subjected to Masson's Trichrome staining to examine the arrangement of collagen fibers. Specifically, the tissue slides were dehydrated in 100% ethanol for 3 minutes two times and then dehydrated in 95% ethanol for 3 minutes. Next, the dehydrated sections were fixed in Bouin's fixing solution overnight at room temperature, and the slides were rinsed with running tap water and stained with Weigert's iron hematoxylin working solution for 10 minutes. After staining, the slides were rinsed with water for 10 minutes, then Biebrich scarlet-acid fuschin solution was added for 5 minutes, and the samples were placed in a phosphomolybdic-phosphotungstic acid solution for 10 minutes. Then, the samples were stained with aniline blue solution for 5 minutes and rinsed with 1% acetic acid for 1 minute. Finally, the sections were mounted with mounting solution. In Masson's trichrome staining, collagen fibers are stained blue and cytoplasm is stained red. Muscle fibers and cell nuclei were stained light pink and dark brown, respectively, and randomly selected regions were photographed, which were observed under an optical microscope at 20X magnification.

As a result of H&E staining and Trichrome staining, it has been found that the density of collagen fibers is significantly decreased and collagen fibers are arranged in parallel in the MOF-containing silicone patch attachment group (test group). It has been found that collagen fibers in the negative control have a high density and arranged out of order. As illustrated in FIGS. 6A and 6B, there is a difference in epidermal thickness between the negative control, the positive control, and the test group (the negative control is Control (Group 1) in FIG. 6A, the positive control is Positive (Group 2) in FIG. 6A, and the test group is MOF treated (Group 3) in FIG. 6A). In particular, the epidermal thickness in the positive control (MOF 0% in FIG. 6B) is decreased compared to that in the negative control (control in FIG. 6B) and the epidermal thickness in the test group (MOF 0.2% in FIG. 6B) is decreased by 49.5% compared to that in the negative control.

FIG. 6C is the results of Masson's Trichrome staining, and it has been confirmed that the collagen fibers have a greatly high density and are arranged in a non-aligned form in the negative control (Control (Group 1) in FIG. 6C, control in FIG. 6D). Compared to that in the negative control, the collagen density in the positive control (Positive (Group 2) in FIG. 6C, MOF 0% in FIG. 6D) is decreased by 7.5% and the collagen density in the test group (MOF treated (Group 3) in FIG. 6C, MOF 0.2% in FIG. 6D) to which a silicone patch containing MOF is attached is decreased by 15.7%, and it has been thus confirmed that the decrease in collagen density in the test group is about 2 times or more that in the positive control. It has been confirmed that the texture of the collagen fibers in the test group is soft and there is a great difference in texture between the test group and the negative control and positive control.

Through this, it has been found that the silicone patch containing MOF according to one aspect of the present invention has a superior effect of healing wounds, specifically hypertrophic scars, such as smooth formation of collagen fibers and decreases in epidermal thickness, compared to a silicone patch not containing MOF.

**[Experimental Example 4] Measurement of expression level of α-SMA, collagen I and TGF-β**

From Experimental Example 3, it has been confirmed that the silicone patch containing MOF has an excellent wound healing effect by decreasing the epidermal thickness and collagen density, and in order to further understand the mechanism of this, Western blot was performed to analyze the expression of myofibroblast biomarker proteins such as α-SMA, collagen I, and TGF-β. At this time, the molecular weights of α-SMA, collagen I, and TGF-β are about 44 kDa, 35 kDa, and 25 kDa, respectively.

The tissue obtained in Experimental Example 2-1 was lysed with a cell lysis buffer and subjected to Western blot analysis according to standard protocols (Yang and Mahmood) [37]. Protein analysis was performed by standard BCA methods, and protein samples were prepared for use in immunoblotting. Mouse anti-α-SMA (1:300; Abcam; cat. no. ab7817), anti-GADPH (1:1000; Santa Cruz Biotechnology; cat. no. sc32233), mouse anti-collagen I (1:1000; Abcam; cat. no. ab90395), and mouse anti-TGF-β1 (1:1000 Abcam; cat. no. ab190503) were used as primary antibodies, and goat anti-mouse IGG H&L (1:5000; Abcam; cat. no. ab6789) was used as a secondary antibody. Images were taken using Amersham Imager 600 (GE Healthcare), and densitometric analysis was performed using Image Quant TL software. The expression of the target proteins was measured using Image J software (National Institutes of Health, Bethesda, MD, USA) and normalized to GAPDH (molecular weight of about 36 kDa) expression, and the results are as illustrated in FIGS. 7A and 7B.

As illustrated in FIGS. 7A and 7B, in addition to GAPDH, α-SMA, collagen I, and TGF-β are most highly expressed in the negative control (Control in FIGS. 7A and 7B) and are further decreased in the positive control (Positive in FIG. 7A, MOF 0% in FIG. 7B) and the test group (MOF in FIG. 7A, MOF 0.2% in FIG. 7B). This suggests that the expression of the biomarker proteins is reduced in a rabbit model to which a silicone patch containing MOF is attached, and the expression of the biomarker proteins (α-SMA, collagen I, and TGF-β) of myofibroblasts is clearly reduced in the test group to which a silicone patch containing MOF is attached as illustrated in FIG. 7B.

Considering that the degree of decrease in biomarker expression is slight in the negative control that undergone no treatment, the rates of decrease in expression of each biomarker protein in the positive control and test group based on that in the negative control are as presented in Table 1 below.

[Table 1]

| Unit: % | | |
|---|---|---|
| Marker protein | Positive control | Test group |
| TGF-β1 | 6.7 | 20 |
| Collagen I (Col-I) | 12.5 | 28 |
| α-SMA | 25.4 | 40 |

As presented in Table 1, the expression level of collagen I is decreased by 12.5% in the positive control and by 28% in the test group compared to that in the negative control, and the expression level of α-SMA is decreased by 25.4% in the positive control and by 40% in the test group compared to that in the negative control ($p < 0.05$). Similarly, the expression level of TGF-β is decreased by 6.7% in the positive control and by 20% in the test group based on that in the negative control. In other words, it has been confirmed that the decreases in expression levels of biomarker proteins (α-SMA, collagen I, and TGF-β) of myofibroblasts in the test group to which a silicone patch containing MOF is attached are about 2 times or more those in the positive control to which a silicone patch not containing MOF is attached.

Overall, it has been found that the silicone patch containing a metal-organic framework according to Example of the present invention has wound healing properties and can be thus utilized as a highly functional wound healing patch or artificial skin.

**Industrial Applicability**

The patch, silicone patch or artificial skin according to one aspect and other aspects of the present invention has an excellent wound healing effect and can be used as a patch, in particular a silicone patch for cell regeneration or skin wound healing, and furthermore, can be used as artificial skin.

**Claims**

1. A patch, which is a silicone patch, or an artificial skin, comprising a metal-organic framework (MOF) and a silicone composition, wherein the metal-organic framework contains one or more metal ions selected from the group consisting of Al and Zr and one or more organic ligands selected from the group consisting of 4,4'-biphenyldicarboxilic acid, benzene-1,4-dicarboxylic acid, 9,10-anthracenedicarboxylic acid, biphenyl-3,3,5,5'-tetracarboxylic acid, biphenyl-3,4',5-tricarboxylic acid, 5-bromoisophthalic acid, 5-cyano-1,3-benzenedicarboxylic acid, 2,2-diamino-4,4'-stilbenedicarboxylic acid, 2,5-diaminoterephthalic acid, 1,1,2,2-tetra(4-carboxylphenyl)ethylene, 2,5-dihydroxyterephthalic acid, 2,2-dinitro-4,4-stilbenedicarboxylic acid, 5-ethynyl-1,3-benzenedicarboxylic acid, 2-hydroxyterephthalic acid, 2,6-naphthalenedicarboxylic acid, 1,2,4,5-tetrakis(4-carboxyphenyl)benzene, 4,4,4"-s-triazine-2,4,6-triyl-tribenzoic acid, 1,3,5-tricarboxybenzene, 1,4,7,10-tetraazacyclododecane-N,N',N",N"'-tetraacetic acid, 1,3,5-tris(4-carboxy[1,1'-biphenyl]-4-yl)benzene, and 1,3,5-triscarboxyphenylethynylbenzene.

**2.** The patch or the artificial skin according to claim 1, which is for skin cell regeneration.

**3.** The patch or the artificial skin according to claim 1 or 2, which is for wound healing.

**4.** The patch or the artificial skin according to claims 1 to 3, wherein the metal-organic framework is MOF-808 (Zr6O4(OH)4(BTC)2(HCOO)6).

**5.** The patch or the artificial skin according to claims 1 to 3, wherein the metal-organic framework is contained at 0.01% to 10% by weight based on a total weight of the metal-organic framework and the silicone composition.

**6.** The patch or the artificial skin according to claims 1 to 5, wherein the metal-organic framework is dispersed in the silicone composition.

**7.** The patch or the artificial skin according to claims 1 to 6, wherein the wound is any one or more selected from the group consisting of cuts, non-healing traumatic wounds, tissue destruction by irradiation, burns, abrasions, lacerations, avulsion wounds, penetrating wounds, gunshot wounds, incisions, burns, frostbite, skin ulcers, dry skin, keratosis, cracking, tearing, dermatitis, surgical or vascular disease wounds, bruises, corneal wounds, decubitus ulcers, bedsore, chronic ulcers, postoperative suture sites, spinal injury wounds, gynecological wounds, chemical wounds, acne and hypertrophic scars.

**8.** The patch or the artificial skin according to claims 1 to 7, wherein the patch has one or more of the following characteristics:

(i) a decrease in hypertrophic scar (HS) at a wound site by 25% to 75% after attachment of the patch compared to HS before attachment;
(ii) a decrease in scar elevation index (SEI) at a wound site by 30% to 40% after attachment of the patch compared to SEI before attachment;
(iii) a decrease in epidermal thickness at a wound site by 25% to 75% after attachment of the patch compared to the epidermal thickness before attachment;
(iv) a decrease in density of collagen fibers at a wound site by 10% to 30% after attachment of the patch compared to the density of collagen fibers before attachment;
(v) a decrease in expression level of $\alpha$-smooth muscle action ($\alpha$-SMA) at a wound site by 30% to 50% after attachment of the patch compared to $\alpha$-SMA before attachment;
(vi) a decrease in expression level of collagen I (Col-I) at a wound site by 20% to 40% after attachment of the patch compared to the expression level of Col-I before attachment; and
(vii) a decrease in expression level of transforming growth factor-$\beta$ (TGF-$\beta$) at a wound site by 10% to 30% after attachment of the patch compared to the expression level of TGF-$\beta$ before attachment.

## Patentansprüche

**1.** Pflaster, das ein Silikonpflaster ist, oder künstliche Haut, umfassend ein metallorganisches Gerüst (MOF) und eine Silikonzusammensetzung, wobei das metallorganische Gerüst ein oder mehrere Metallionen, ausgewählt aus der Gruppe bestehend aus Al und Zr ist/sind, und einen oder mehrere organische Liganden, ausgewählt aus der Gruppe bestehend aus 4,4'-Biphenyldicarbonsäure, Benzol-1,4-dicarbonsäure, 9,10-Anthracendicarbonsäure, Biphenyl-3,3,5,5'-tetracarbonsäure, Biphenyl-3,4',5-tricarbonsäure, 5-Bromisophthalsäure, 5-Cyano-1,3-benzoldicarbonsäure, 2,2-Diamino-4,4'-stilbendicarbonsäure, 2,5-Diaminoterephthalsäure, 1,1,2,2,-Tetra-(4-carboxylphenyl) ethylen, 2,5-Dihydroxyterepthalsäure, 2,2-Dinitro-4,4-stilbendicarbonsäure, 5-Ethinyl-1,3-benzoldicarbonsäure, 2-Hydroxyterephthalsäure, 2,6-Naphthalindicarbonsäure, 1,2,4,5-Tetrakis-(4-carboxyphenyl)benzol, 4,4,4"-s-Triazin-2,4,6-triyltribenzoesäure, 1,3,5-Tricarboxybenzol, 1,4,7,10-Tetraazacyclododecan-N,N',N",N‴-tetraessigsäure, 1,3,5-Tris-(4-carboxy[1,1'-biphenyl]-4-yl)benzol und 1,3,5-Triscarboxyphenylethinylbenzol.

**2.** Pflaster oder künstliche Haut nach Anspruch 1, das bzw. die zur Hautzellregeneration ist.

3. Pflaster oder künstliche Haut nach Anspruch 1 oder 2, das bzw. die zur Wundheilung ist.

4. Pflaster oder künstliche Haut nach einem der Ansprüche 1 bis 3, wobei das metallorganische Gerüst MOF-808 (Zr6O4(OH)4(BTC)2(HCOO)6) ist.

5. Pflaster oder künstliche Haut nach einem der Ansprüche 1 bis 3, wobei das metallorganische Gerüst in 0,01 Gew.-% bis 10 Gew.-%, basierend auf einem Gesamtgewicht des metallorganischen Gerüsts und der Silikonzusammensetzung, enthalten ist.

6. Pflaster oder künstliche Haut nach einem der Ansprüche 1 bis 5, wobei das metallorganische Gerüst in der Silikonzusammensetzung verteilt ist.

7. Pflaster oder künstliche Haut nach einem der Ansprüche 1 bis 6, wobei die Wunde irgendeine oder mehrere, ausgewählt aus der Gruppe bestehend aus Schnittwunden, nichtheilenden traumatischen Wunden, Gewebezerstörung aufgrund von Bestrahlung, Verbrennungen, Abschürfungen, Platzwunden, Abrisswunden, penetrierenden Wunden, Schussverletzungen, Inzisionen, Verbrennungen, Erfrierungen, Hautgeschwüren, trockener Haut, Keratose, Rissen, Reißen, Dermatitis, chirurgischen oder Gefäßerkrankungswunden, Prellungen, kornealen Wunden, Druckgeschwüren, wundgelegenen Stellen, chronischen Geschwüren, postoperativen Nahtstellen, Wirbelsäulenverletzungswunden, gynäkologischen Wunden, chemischen Wunden, Akne und hypertrophen Narben ist/sind.

8. Pflaster oder künstliche Haut nach einem der Ansprüche 1 bis 7, wobei das Pflaster ein oder mehrere der folgenden Charakteristika hat:

(i) eine Verringerung von hypertrophen Narbe (HS) an einer Wundstelle um 25 % bis 75 % nach Aufbringen des Pflasters verglichen mit der HS vor dem Aufbringen;
(ii) eine Verringerung eines Narbenerhebungsindexes (SEI) an einer Wundstelle um 30 % bis 40 % nach Aufbringen des Pflasters verglichen mit dem SEI vor dem Aufbringen;
(iii) eine Verringerung einer epidermalen Dicke an einer Wundstelle um 25 % bis 75 % nach Aufbringen des Pflasters verglichen mit der epidermalen Dicke vor dem Aufbringen;
(iv) eine Verringerung einer Dichte von Kollagenfasern an einer Wundstelle um 10 % bis 30 % nach Aufbringen des Pflasters verglichen mit der Dichte von Kollagenfasern vor dem Aufbringen;
(v) eine Verringerung eines Expressionsniveaus von $\alpha$-Glattmuskelaktin (SMA) an einer Wundstelle um 30 % bis 50 % nach Aufbringen des Pflasters verglichen mit der $\alpha$-SMA vor dem Aufbringen;
(vi) eine Verringerung eines Expressionsniveaus von Kollagen-I (Kol-I) an einer Wundstelle um 20 % bis 40 % nach Aufbringen des Pflasters verglichen mit dem Expressionsniveau von Kol-I vor dem Aufbringen; und und
(vii) eine Verringerung eines Expressionsniveaus von transformierendem Wachstumsfaktor-$\beta$ (TGF-$\beta$) an einer Wundstelle um 10 % bis 30 % nach Aufbringen des Pflasters verglichen mit dem Expressionsniveau von TGF-$\beta$ vor dem Aufbringen.

## Revendications

1. Patch qui est un patch de silicone, ou peau artificielle, comprenant une structure métal-organique (MOF) et une composition de silicone, dans lequel la structure métal-organique contient un ou plusieurs ions métalliques choisis dans le groupe constitué par Al et Zr et un ou plusieurs ligands organiques choisis dans le groupe constitué par l'acide 4,4'-biphényldicarboxylique, l'acide benzène-1,4-dicarboxylique, l'acide 9,10-anthracènedicarboxylique, l'acide biphéhyl-3,3,5,5'-tétracarboxylique, l'acide biphényl-3,4',5-tricarboxylique, l'acide 5-bromoisophtalique, l'acide 5-cyano-1,3-benzènedicarboxylique, l'acide 2,2-diamino-4,4'-stilbènedicarboxylique, l'acide 2,5-diaminotéréphtalique, le 1,1,2,2-tétra(4-carboxyphényl)éthylène, l'acide 2,5-dihydroxytéréphtalique, l'acide 2,2-dinitro-4,4-stilbènedicarboxylique, l'acide 5-éthynyl-1,3-benzènedicarboxylique, l'acide 2-hydroxytéréphtalique, l'acide 2,6-naphtalènedicarboxylique, le 1,2,4,5-tétrakis(4-carboxyphényl)benzène, l'acide 4,4,4"-s-triazine-2,4,6-triyl-tribenzoïque, le 1,3,5-tricarboxybenzène, l'acide 1,4,7,10-tétraazacyclododécane-N,N',N",N'"-tétraacétique, le 1,3,5-tris(4-carboxy [1,1'-biphényl]-4-yl)benzène, et le 1,3,5-tris-carboxyphényléthynylbenzène.

2. Patch ou peau artificielle selon la revendication 1, qui est destiné à la régénération de cellules cutanées.

3. Patch ou peau artificielle selon la revendication 1 ou 2, qui est destiné à la cicatrisation de plaies.

**4.** Patch ou peau artificielle selon les revendications 1 à 3, dans lequel la structure métal-organique est MOF-808 ($Zr_6O_4(OH)_4(BTC)_2(HCCO)_6$).

**5.** Patch ou peau artificielle selon les revendications 1 à 3, dans lequel la structure métal-organique est contenue à raison de 0,01 à 10 % en poids par rapport au poids total de la structure métal-organique et de la composition de silicone.

**6.** Patch ou peau artificielle selon les revendications 1 à 5, dans lequel la structure métal-organique est dispersée dans la composition de silicone.

**7.** Patch ou peau artificielle selon les revendications 1 à 6, dans lequel la plaie est une ou plusieurs quelconques choisies dans le groupe constitué par les coupures, les plaies traumatiques ne cicatrisant pas, une destruction tissulaire par irradiation, les brûlures, les abrasions, les lacérations, les plaies par avulsion, les plaies pénétrantes, les blessures par arme à feu, les incisions, les brûlures, les engelures, les ulcères cutanés, une sécheresse cutanée, une kératose, une fissuration, une déchirure, une dermatite, les plaies chirurgicales ou d'origine vasculaire, les ecchymoses, les lésions cornéennes, les ulcères de décubitus, les escarres, les ulcères chroniques, les sites de suture postopératoires, les plaies associées à des lésions médullaires, les plaies gynécologiques, les lésions chimiques, l'acné, et les cicatrices hypertrophiques.

**8.** Patch ou peau artificielle selon les revendications 1 à 7, dans lequel le patch a une ou plusieurs des caractéristiques suivantes :

(i) une diminution des cicatrices hypertrophiques (HS) au niveau d'un site de lésion de 25 % à 75 % après attachement du patch, en comparaison avec les HS avant attachement ;
(ii) une diminution de l'indice d'élévation de cicatrice (SEI) au niveau d'un site de lésion de 30 % à 40 % après attachement du patch, en comparaison avec le SEI avant attachement ;
(iii) une diminution de l'épaisseur de l'épiderme au niveau d'un site de lésion de 25 % à 75 % après attachement du patch, en comparaison avec l'épaisseur de l'épiderme avant attachement ;
(iv) une diminution de la densité des fibres de collagène au niveau d'un site de lésion de 10 % à 30 % après attachement du patch, en comparaison avec la densité des fibres de collagène avant attachement ;
(v) une diminution du niveau d'expression de l'actine musculaire lisse alpha (a-SMA) au niveau d'un site de lésion de 30 % à 50 % après attachement du patch, en comparaison avec celui de l'a-SMA avant attachement ;
(vi) une diminution du niveau d'expression du collagène I (Col-I) au niveau d'un site de lésion de 20 % à 40 % après attachement du patch, en comparaison avec le niveau d'expression du Col-I avant attachement ; et
(vii) une diminution du niveau d'expression du facteur de croissance transformant bêta (TGF-$\beta$) au niveau d'un site de lésion de 10 % à 30 % après attachement du patch, en comparaison avec le niveau d'expression du TGF-$\beta$ avant attachement.

FIG. 1A

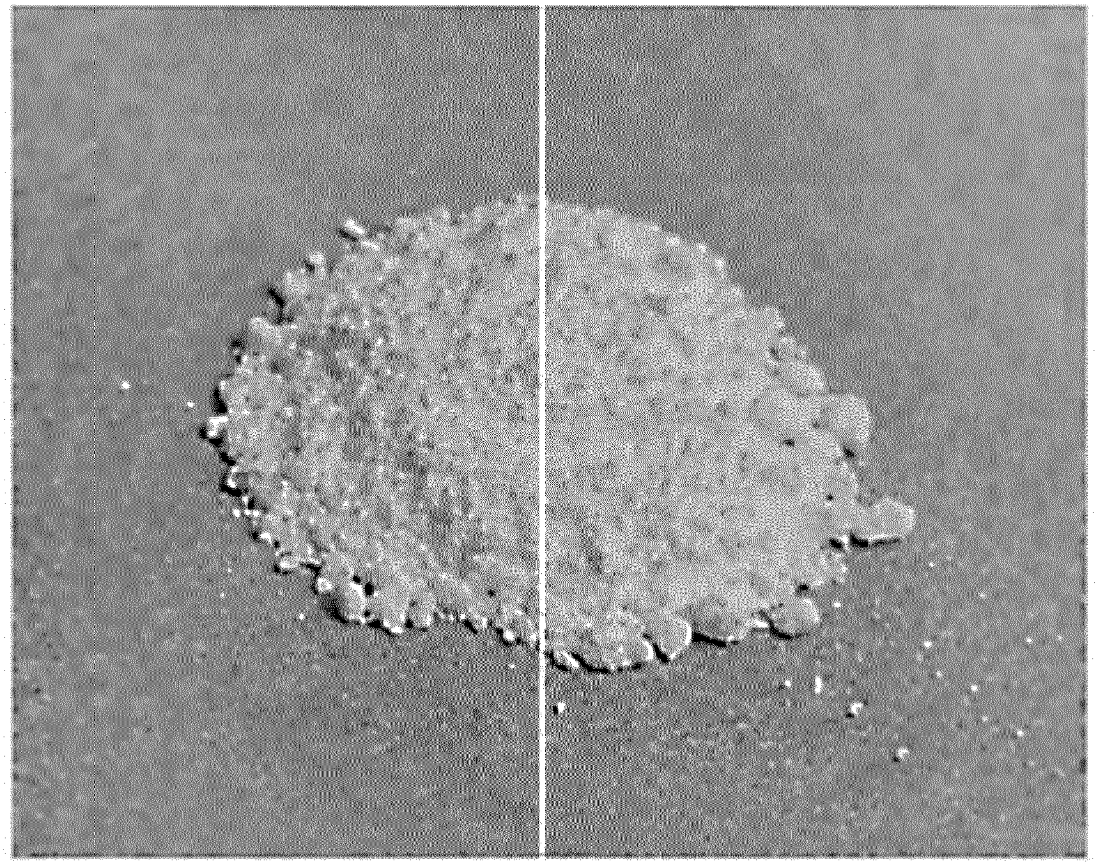

FIG. 1B

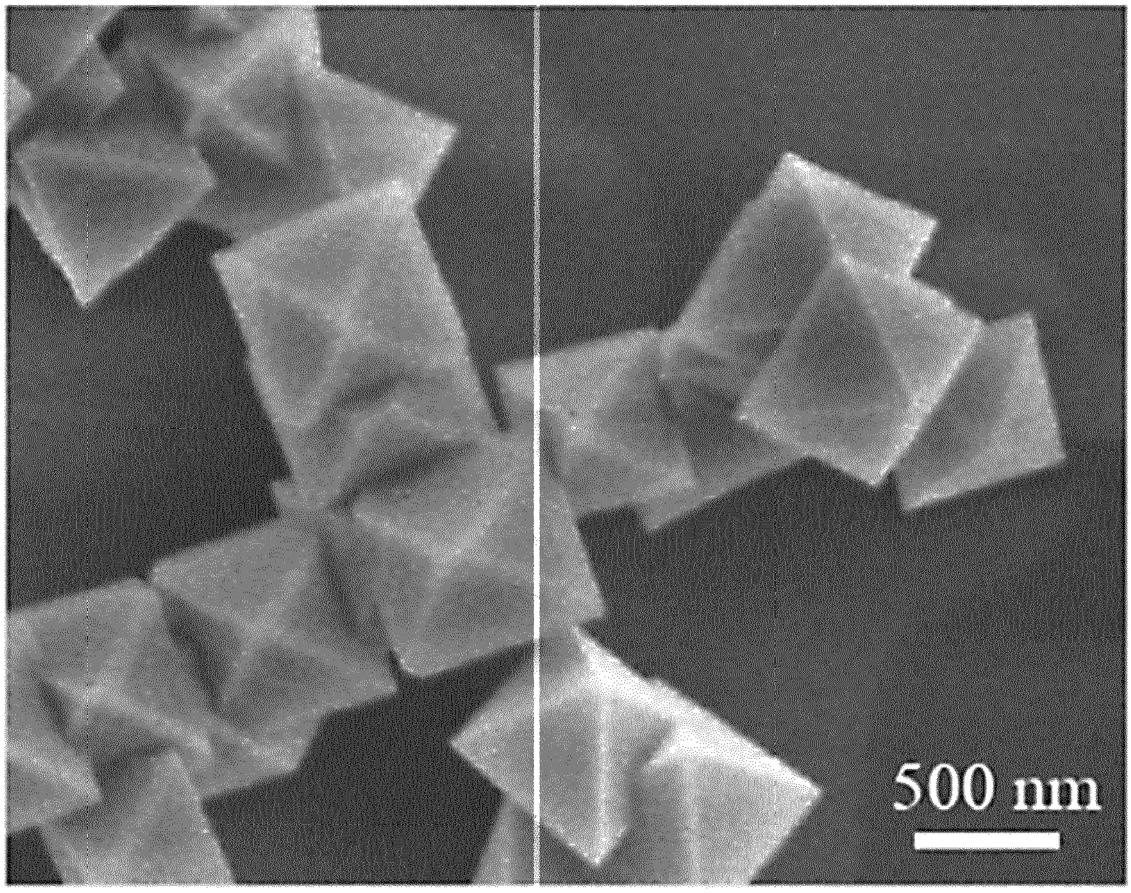

FIG. 1C

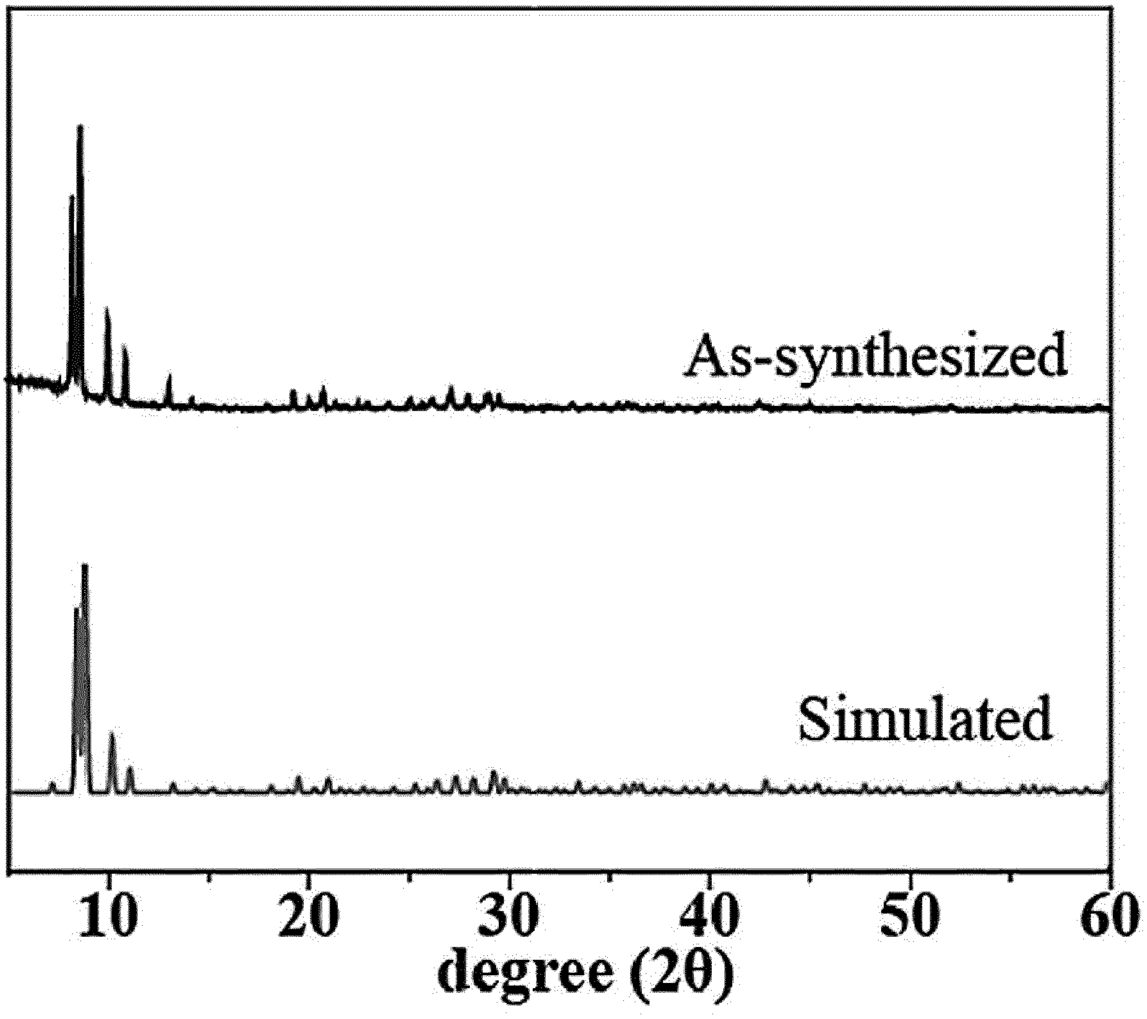

FIG. 1D

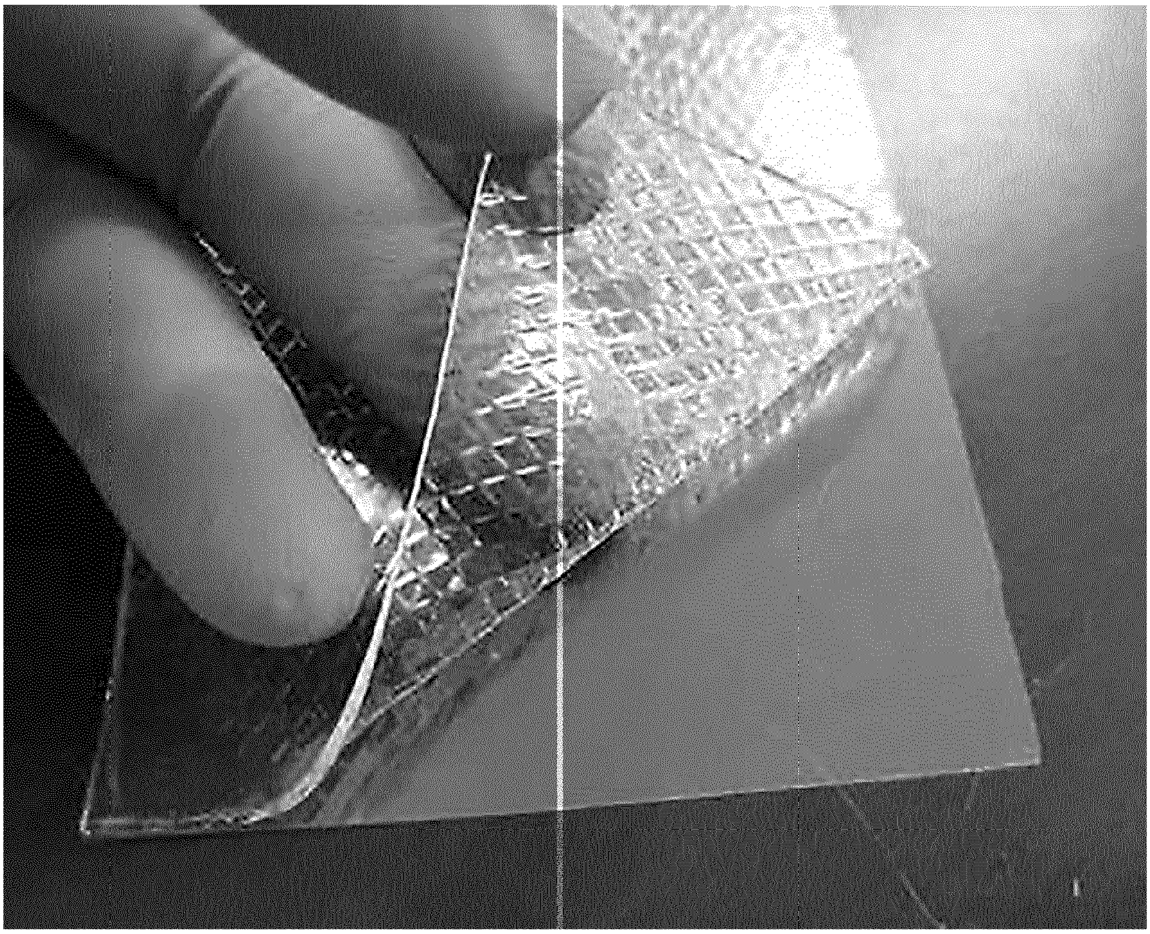

FIG. 1E

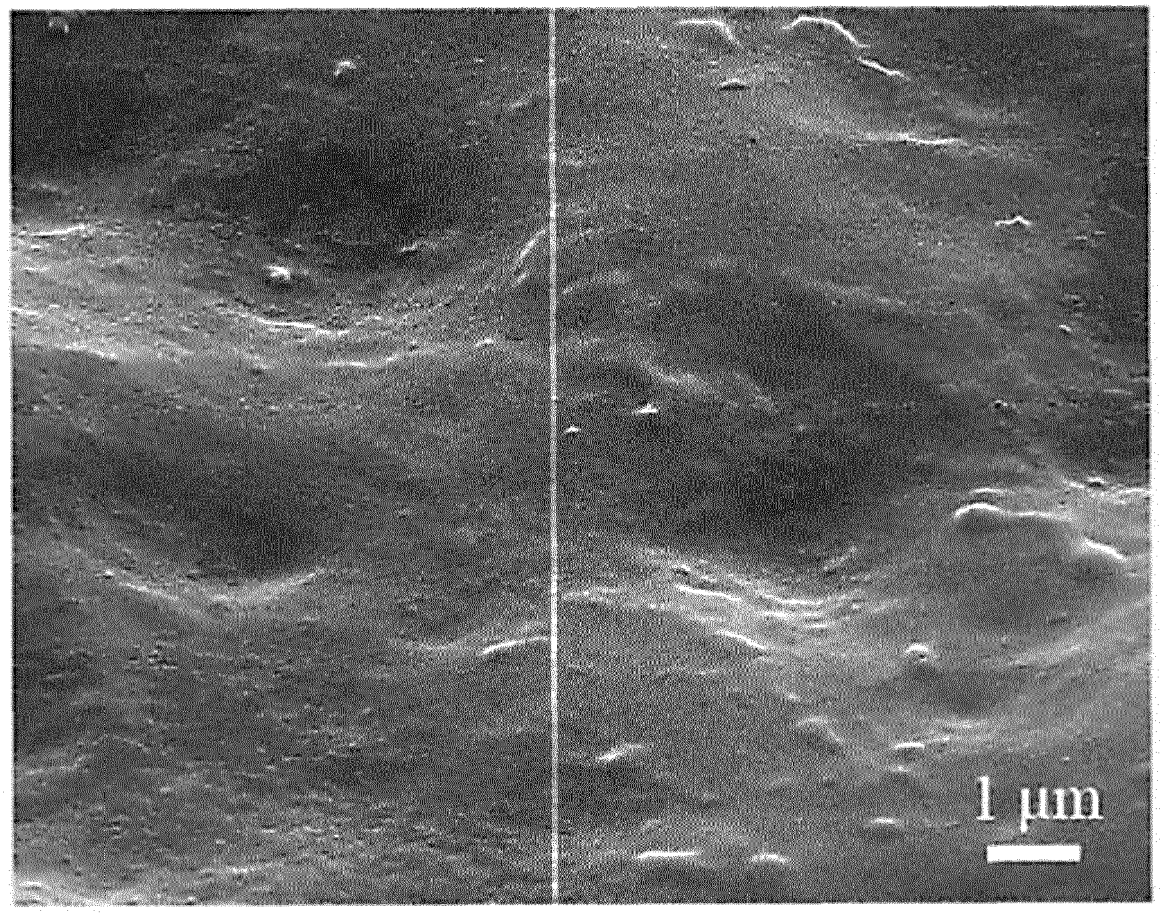

FIG. 1F

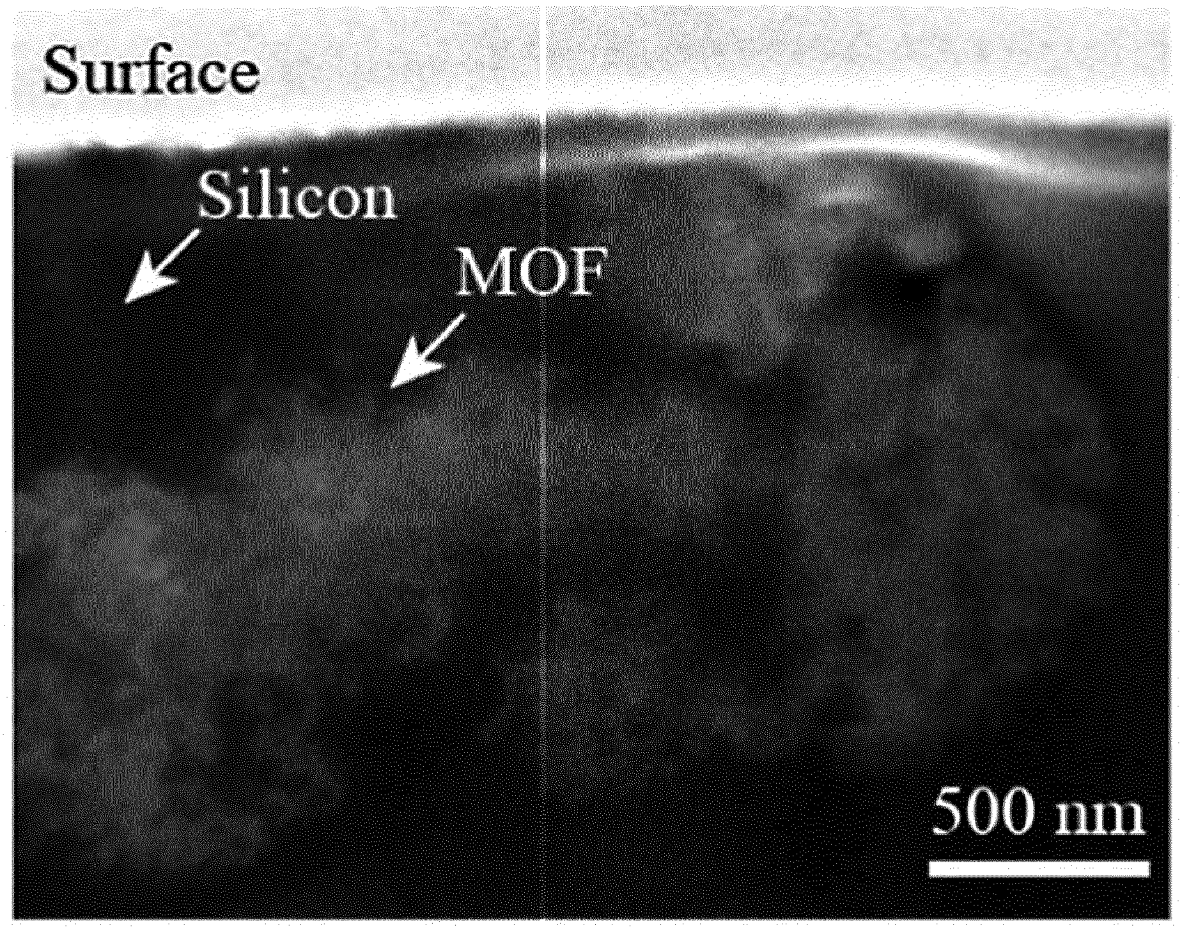

FIG. 2A

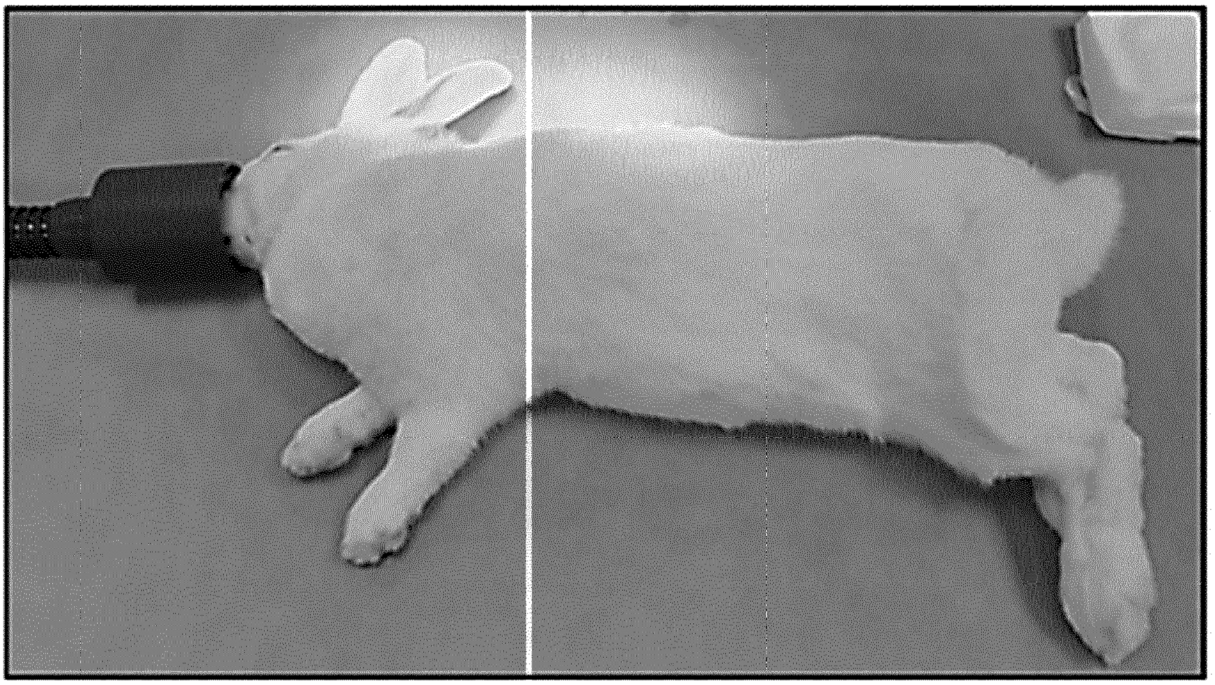

# FIG. 2B

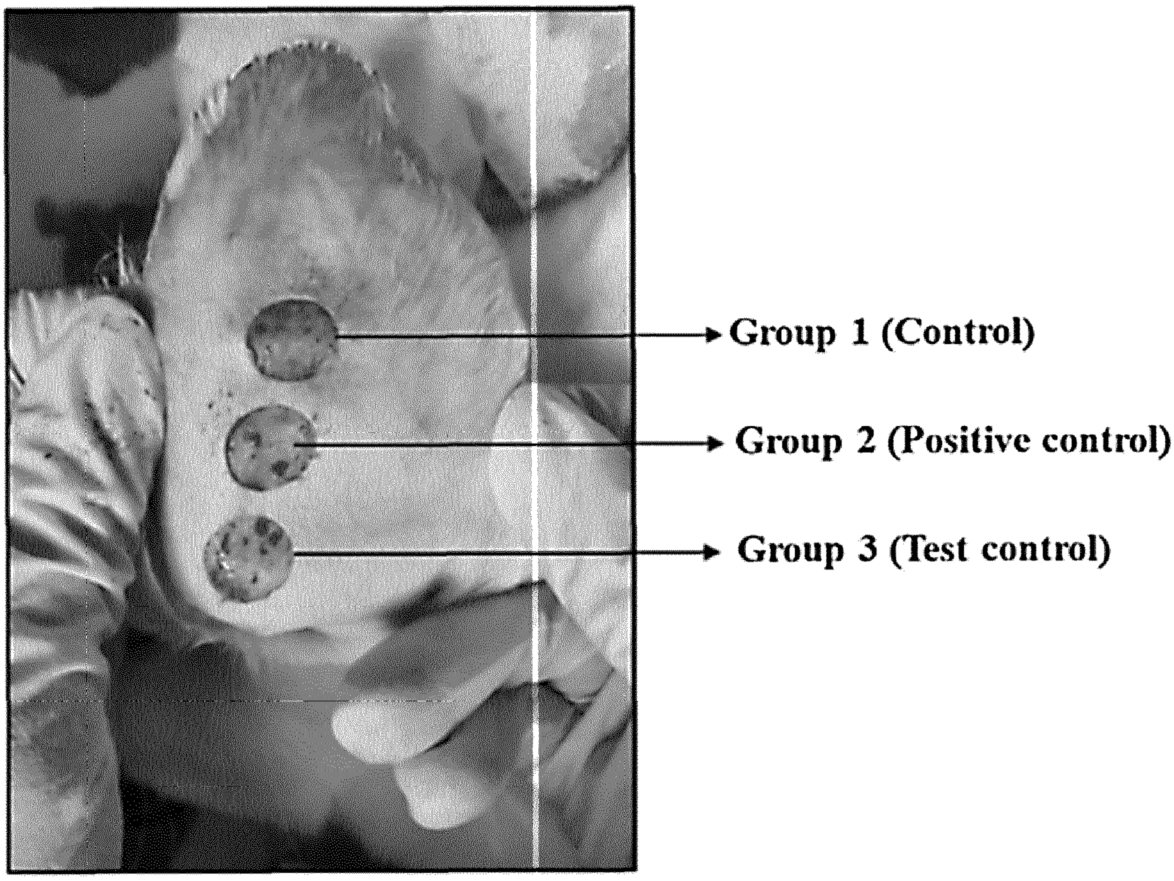
Group 1 (Control)
Group 2 (Positive control)
Group 3 (Test control)

FIG. 3A

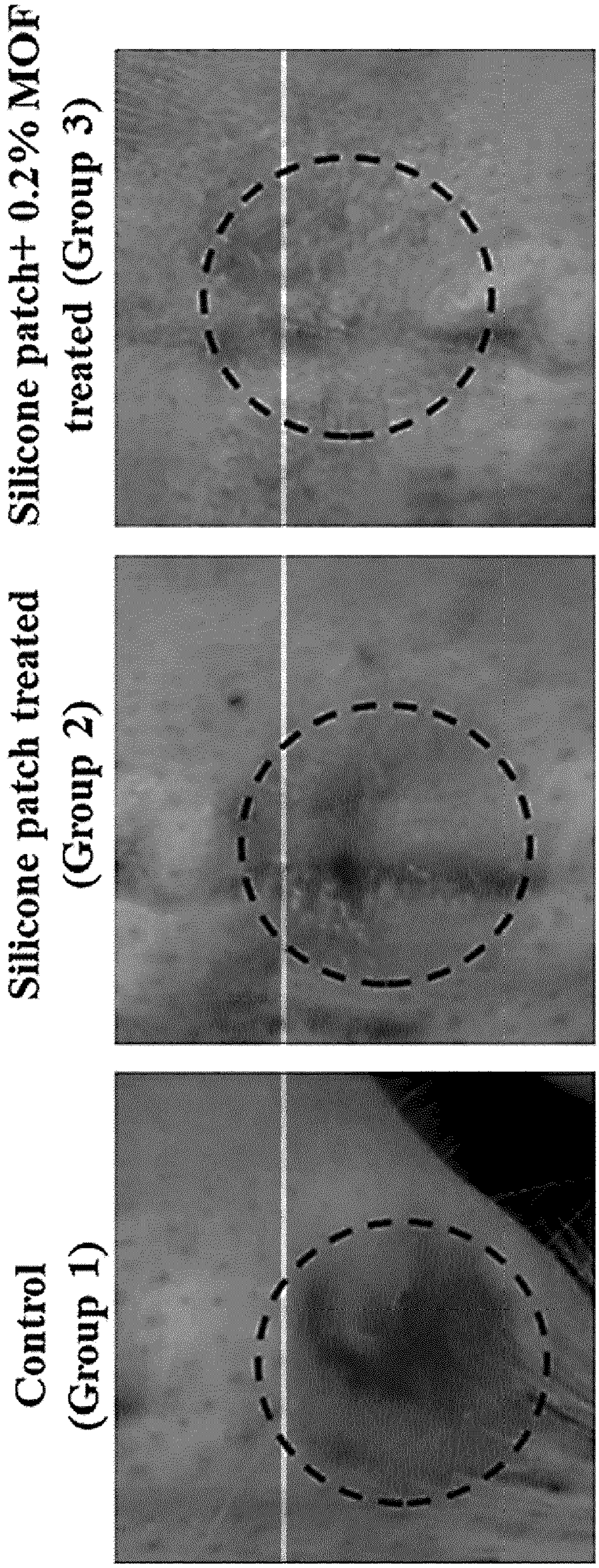
Control
(Group 1)
Silicone patch treated
(Group 2)
Silicone patch+ 0.2% MOF
treated (Group 3)

# FIG. 3B

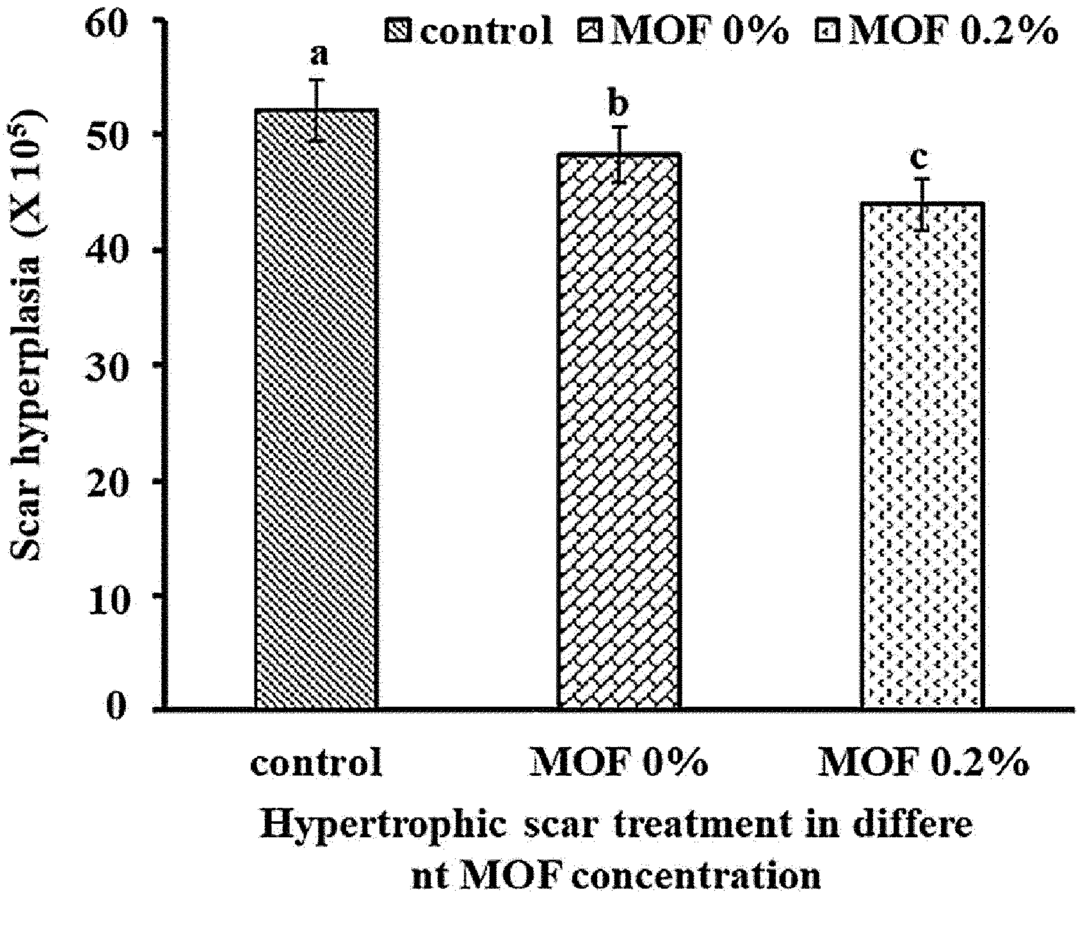
60
50
40
30
20
10
0
Scar hyperplasia (X 10⁵)
control
MOF 0%
MOF 0.2%
a
b
c
control
MOF 0%
MOF 0.2%
Hypertrophic scar treatment in differe
nt MOF concentration

FIG. 4A

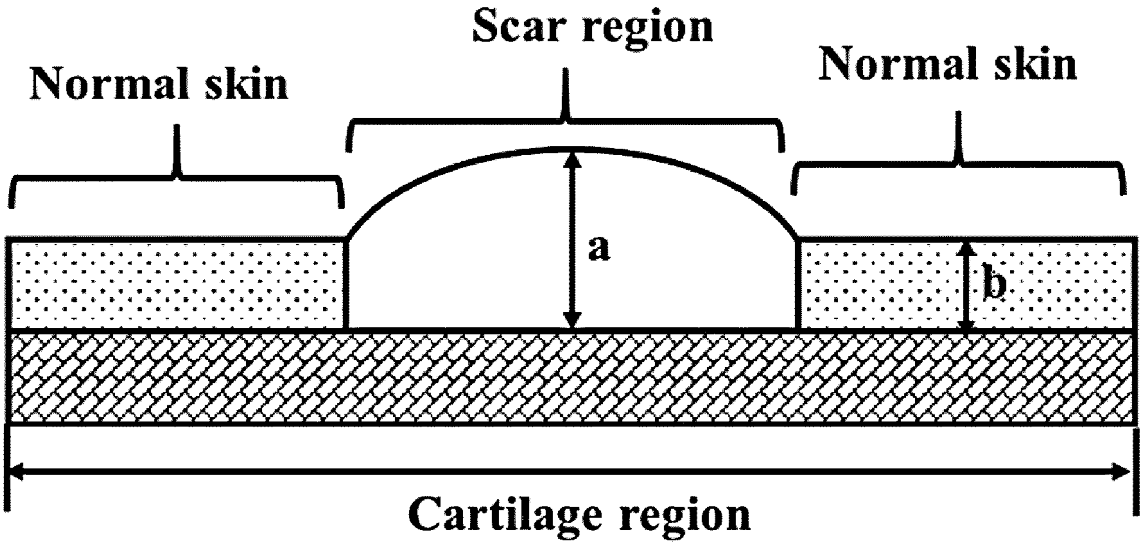

FIG. 4B

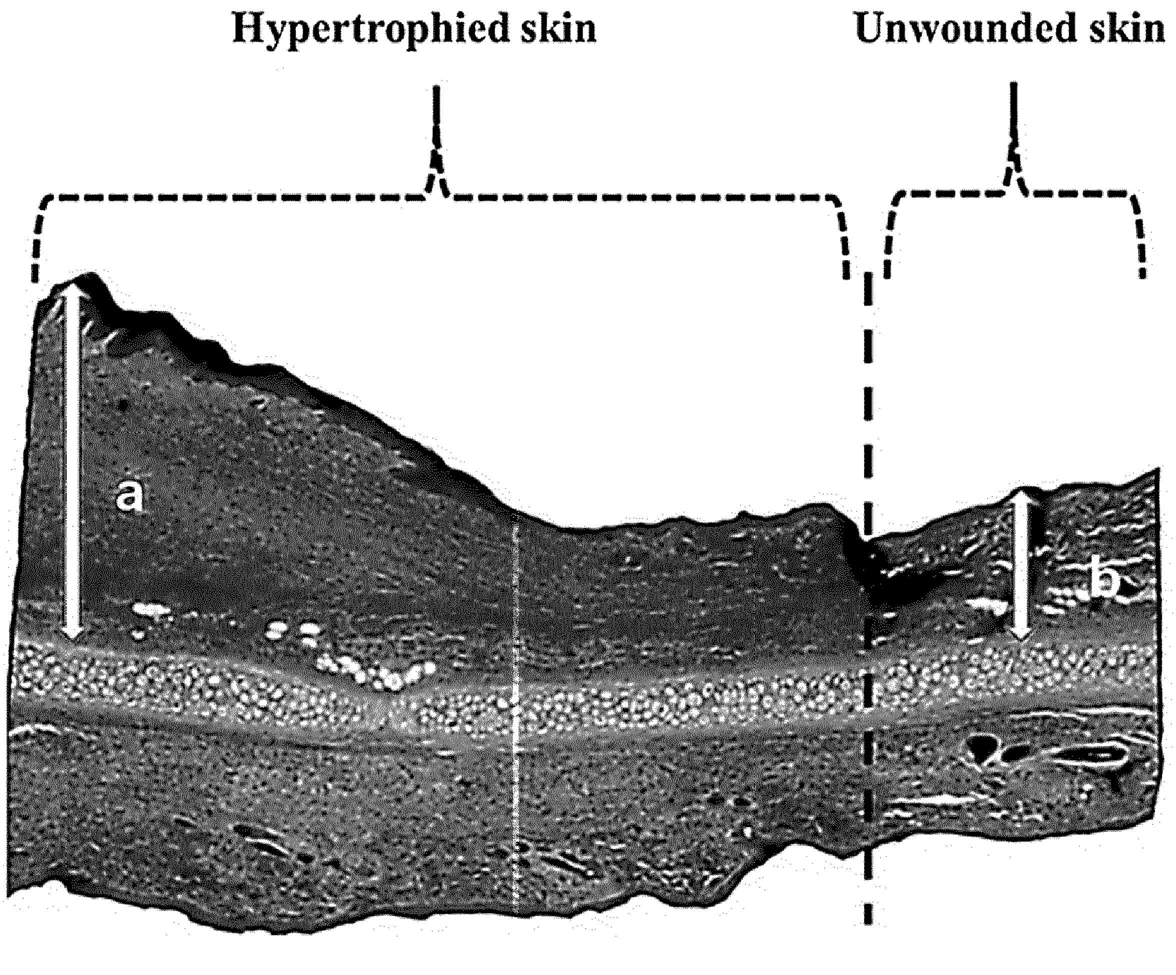

FIG. 5

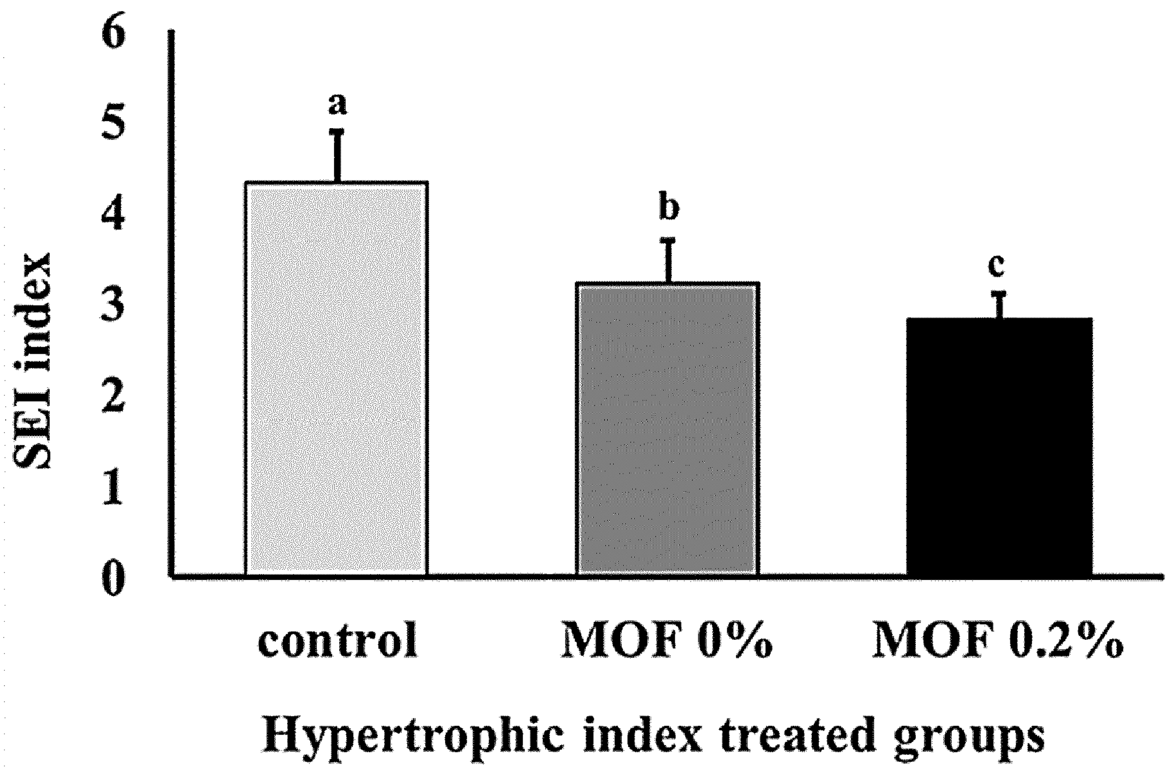
6
5
4
3
2
1
0
SEI index
a
b
c
control
MOF 0%
MOF 0.2%
Hypertrophic index treated groups

FIG. 6A

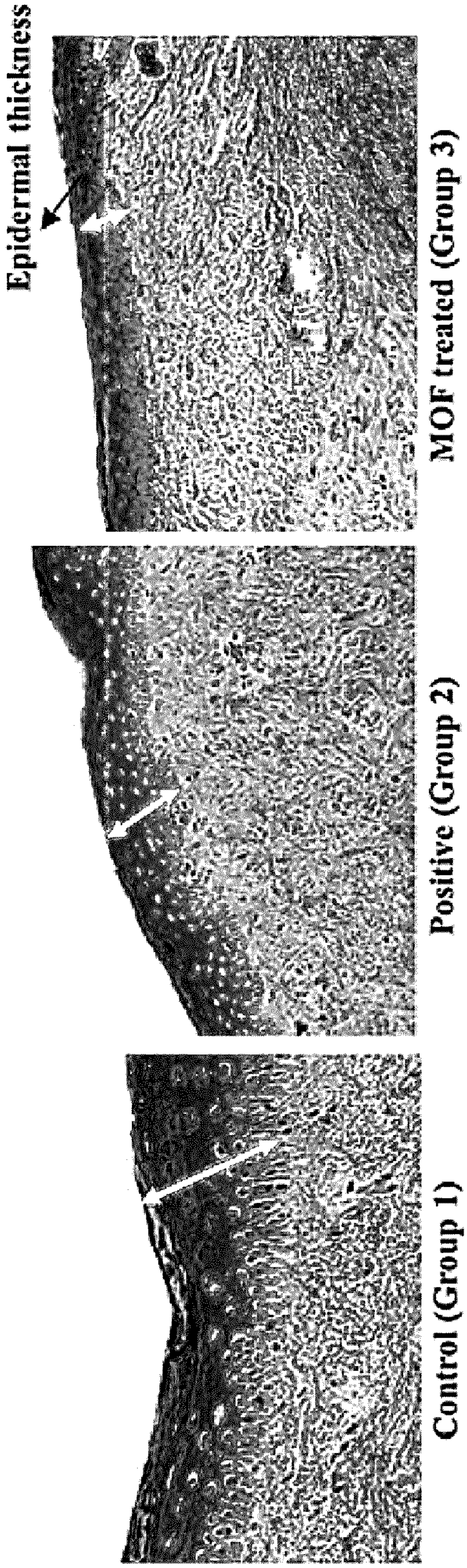
Epidermal thickness
Control (Group 1)
Positive (Group 2)
MOF treated (Group 3)

FIG. 6B

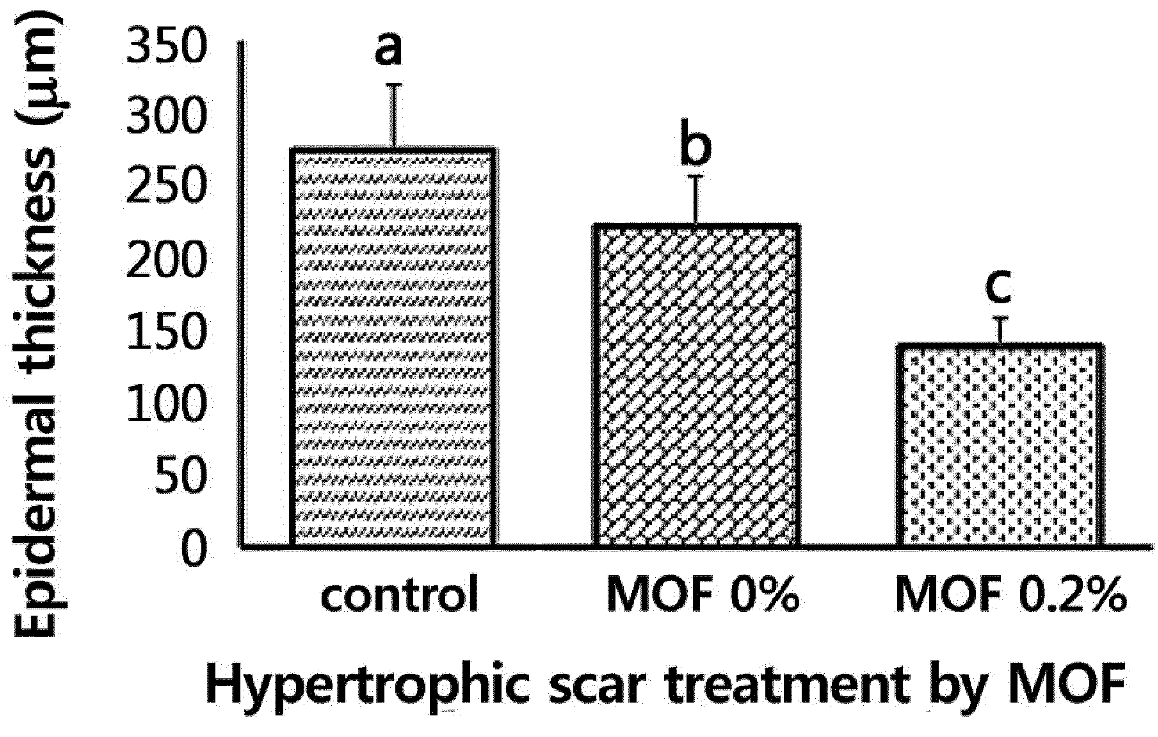
Epidermal thickness (µm)
350
300
250
200
150
100
50
0
a
b
c
control
MOF 0%
MOF 0.2%
Hypertrophic scar treatment by MOF

FIG. 6C

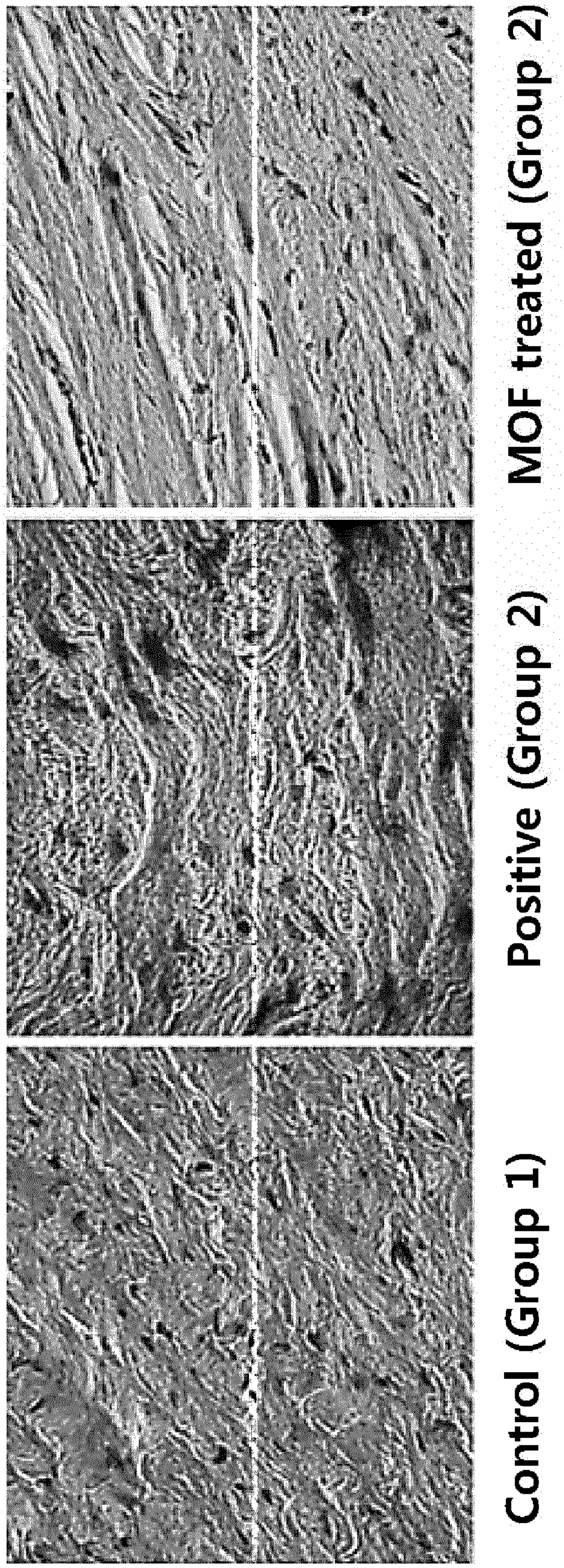

FIG. 6D

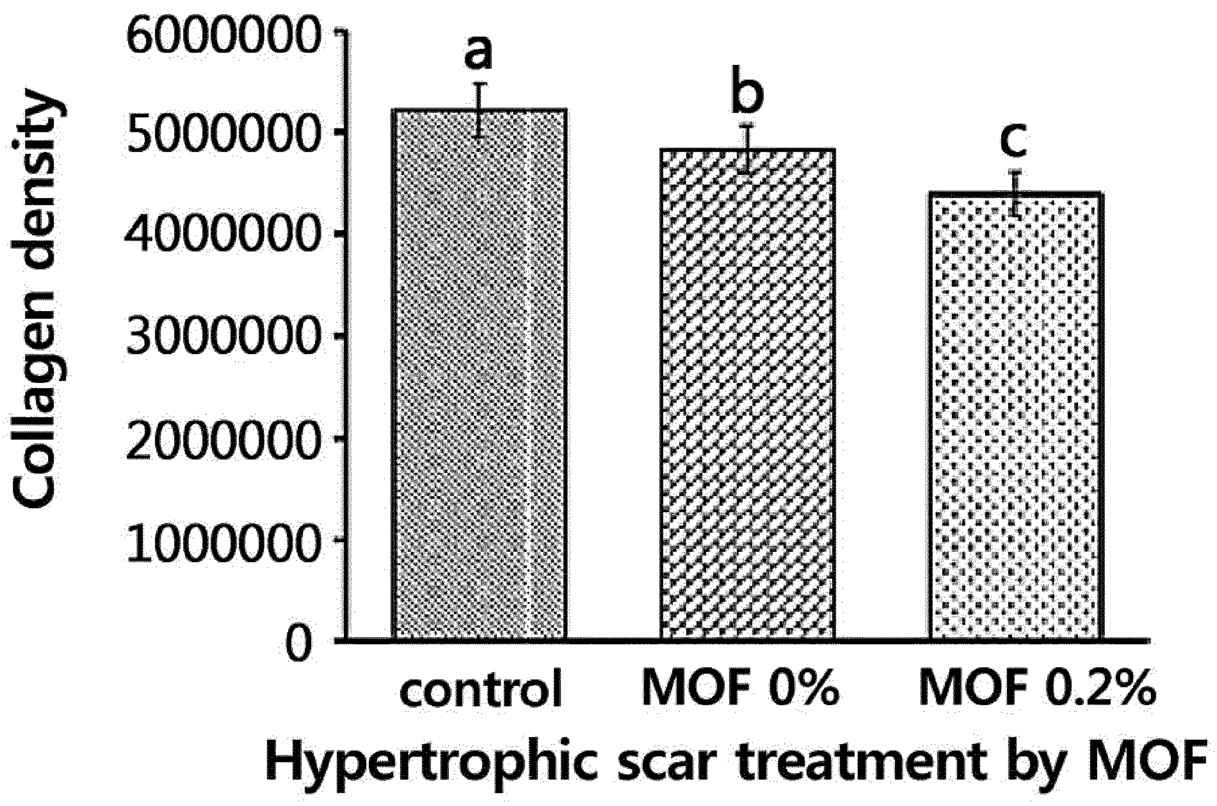
Collagen density
6000000
5000000
4000000
3000000
2000000
1000000
0
a
b
c
control
MOF 0%
MOF 0.2%
Hypertrophic scar treatment by MOF

FIG. 7A

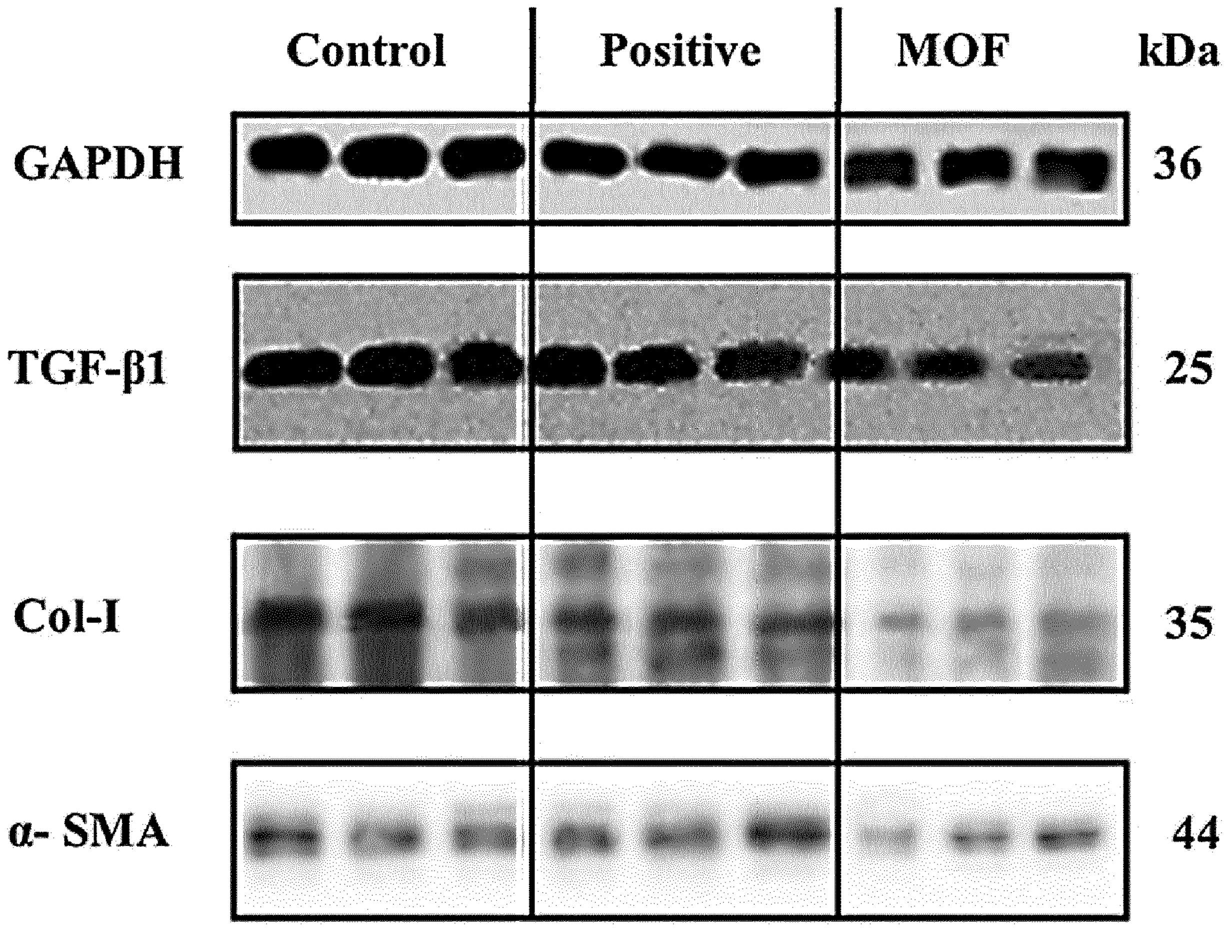

FIG. 7B

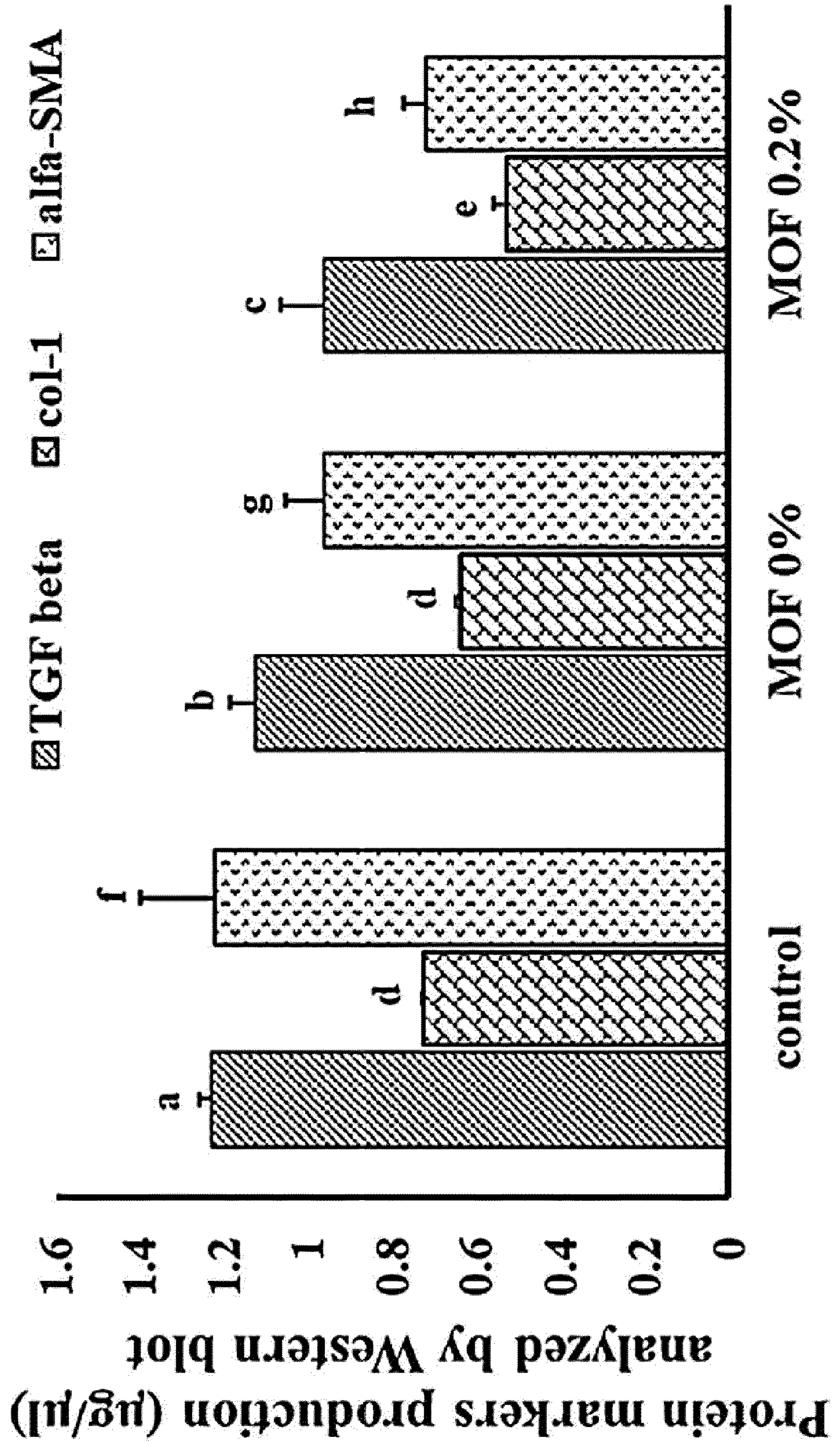
TGF beta
col-1
alfa-SMA
Protein markers production (μg/μl) analyzed by Western blot
0
0.2
0.4
0.6
0.8
1
1.2
1.4
1.6
a
d
f
b
d
g
c
e
h
control
MOF 0%
MOF 0.2%

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019235742 A1
- CN 109111575 A
- CN 1083555166 A
- CN 107823220 A


### Non-patent literature cited in the description


- **G. GAUGLITZ**. Management of keloids and hypertrophic scars: current and emerging options. *Clin. Cosmet. Investig. Dermatol.*, 2013, 103, https://doi.org/10.2147/ccid.s35252
- **A. V SHINDE** ; **C. HUMERES** ; **N.G. FRANGOGIANNIS**. The role of α-smooth muscle actin in fibroblast-mediated matrix contraction and remodeling. *Biochim. Biophys. Acta - Mol. Basis Dis.*, 2017, vol. 1863, 298-309, https://doi.org/10.1016/j.bbadis.2016.11.006
- **M, XUE** ; **C.J. JACKSON**. Extracellular matrix reorganization during wound healing and its impact on abnormal scarring. *Adv. Wound Care.*, 2015, vol. 4 (3), 119-136, https://doi.org/10.1089/wound.2013.0485
- **V. MOULIN** ; **S. LAROCHELLE** ; **C. LANGLOIS** ; **I. THIBAULT** ; **C.A. LOPEZ-VALLE** ; **M. ROY**. Normal skin wound and hypertrophic scar myofibroblasts have differential responses to apoptotic inductors.. *J. Cell Physiol.*, 2004, vol. 198, 350, https://doi.org/10.1002/jcp.10415
- **M. SHAH** ; **D.M. FOREMAN** ; **M.W. FERGUSON**. Neutralisation of TGF-beta 1 and TGF-beta 2 or exogenous addition of TGF-beta 3 to cutaneous rat wounds reduces scarring. *J. Cell Sci.*, 1995, vol. 108 (3), 985-1002
- **A. LEASK**. Focal Adhesion Kinase: A Key Mediator of Transforming Growth Factor Beta Signaling in Fibroblasts. *Adv. Wound Care.*, 2013, vol. 2, 247-249, https://doi.org/10.1089/wound.2012.0363